# EUROPEAN PATENT SPECIFICATION

(11) **EP 3 420 093 B1**
(45) Date of publication and mention of the grant of the patent: **23.11.2022**
(21) Application number: 17708450.6
(22) Date of filing: 24.02.2017
(51) Int. Cl.: C12N 15/87, C12N 15/10, C12N 9/22

(54) **MICELLE BASED SYSTEM NUCLEASE ENCAPSULATION FOR IN-VIVO GENE EDITING**
NUKLEASEVERKAPSELUNG IN EINEM SYSTEM AUF MIZELLENBASIS FÜR IN-VIVO-GENEDITIERUNG
ENCAPSULATION NUCLÉASE DANS UN SYSTÈME À BASE DE MICELLES DESTINÉE À L'ÉDITION DE GÈNE IN VIVO

(30) Priority: 26.02.2016 DK 201670111
(43) Date of publication of application: 02.01.2019
(73) Proprietor: Cellectis, 75013 Paris (FR)
(72) Inventor: DUCHATEAU, Philippe, 11500 Saint Louis et Parahou (FR); ZENNOU, Veronique, Jersey City, NJ 07302 (US)
(74) Representative: Zacco Denmark A/S
(86) International application number: PCT/EP2017/054264
(87) International publication number: WO 2017/144630

(56) References cited:
- WO-A1-2015/089427
- WO-A1-2015/089462
- WO-A1-2016/118697
- WO-A2-2015/092024
- JP-A- 2015 002 723
- KRISTIE BLOOM ET AL: "Inactivation of Hepatitis B Virus Replication in Cultured Cells and In Vivo with Engineered Transcription Activator-Like Effector Nucleases", MOLECULAR THERAPY, vol. 21, no. 10, 25 July 2013 (2013-07-25) , pages 1889-1897, XP055155712, ISSN: 1525-0016, DOI: 10.1038/mt.2013.170
- ANDREW J GEALL ET AL: "Nonviral delivery of self-amplifying RNA vaccines", PROCEEDINGS OF THE NATIONAL ACADEMY OF SCIENCES, NATIONAL ACADEMY OF SCIENCES, US, vol. 109, no. 36, 4 September 2012 (2012-09-04), pages 14604-14609, XP002683929, ISSN: 0027-8424, DOI: 10.1073/PNAS.1209367109 [retrieved on 2012-08-20]
- GENC BASHA ET AL: "Influence of Cationic Lipid Composition on Gene Silencing Properties of Lipid Nanoparticle Formulations of siRNA in Antigen-Presenting Cells", MOLECULAR THERAPY, vol. 19, no. 12, 1 December 2011 (2011-12-01), pages 2186-2200, XP055024764, ISSN: 1525-0016, DOI: 10.1038/mt.2011.190
- SEAN C SEMPLE ET AL: "Rational design of cationic lipids for siRNA delivery", NATURE BIOTECHNOLOGY, NATURE PUBLISHING GROUP, US, vol. 28, no. 2, 1 February 2010 (2010-02-01), pages 172-176, XP002633693, ISSN: 1087-0156, DOI: 10.1038/NBT.1602 [retrieved on 2010-01-17]
- LIU YANG ET AL: "Designer Lipids Advance Systemic siRNA Delivery", MOLECULAR THERAPY, NATURE PUBLISHING GROUP, GB, vol. 18, no. 4, 1 April 2010 (2010-04-01), pages 669-670, XP002684383, ISSN: 1525-0016, DOI: 10.1038/MT.2010.39
- AMBEGIA E ET AL: "Stabilized plasmid-lipid particles containing PEG-diacylglycerols exhibit extended circulation lifetimes and tumor selective gene expression", BIOCHIMICA ET BIOPHYSICA ACTA (BBA) - BIOMEMBRANES, ELSEVIER, AMSTERDAM, NL, vol. 1669, no. 2, 20 May 2005 (2005-05-20) , pages 155-163, XP027733898, ISSN: 0005-2736 [retrieved on 2005-05-20]
- DREYER TIMOTHY ET AL: "Improved antiviral efficacy using TALEN-mediated homology directed recombination to introduce artificial primary miRNAs into DNA of hepatitis B virus", BIOCHEMICAL AND BIOPHYSICAL RESEARCH COMMUNICATIONS, ELSEVIER, AMSTERDAM, NL, vol. 478, no. 4, 30 August 2016 (2016-08-30), pages 1563-1568, XP029732587, ISSN: 0006-291X, DOI: 10.1016/J.BBRC.2016.08.152
- Joshua T. Schiffer ET AL: "Predictors of Hepatitis B Cure Using Gene Therapy to Deliver DNA Cleavage Enzymes: A Mathematical Modeling Approach", PLoS Computational Biology, vol. 9, no. 7, 4 July 2013 (2013-07-04), page e1003131, XP055155732, DOI: 10.1371/journal.pcbi.1003131
- ALINO S F ET AL: "RECOMBINANT CDNA ENCAPSULATION IN SMALL LIPOSOMES WITH HEPATOCYTE ACCESS ABILITY", JOURNAL OF MICROENCAPSULATION, TAYLOR AND FRANCIS, BASINGSTOKE, GB, vol. 10, no. 2, 1 April 1993 (1993-04-01), pages 163-170, XP000303735, ISSN: 0265-2048

## Description

### Field of the invention

The invention pertains to therapies that require gene editing, and more specifically to non-viral methods for *in vivo* delivery of endonuclease reagents to specific tissues or cells. According to the invention, the endonuclease reagents are encapsulated into micelle structures of 50 to 100 nm diameter for intravenous injection. The invention thus provides therapeutic composition including such micelles structures, by which endonuclease reagents can be released into cell under RNA form for their use in the treatment of gene related diseases.

### Background of the invention

The potential of gene editing in various therapies has long been envisioned by the applicant (WO2004067753), especially to repair or modulate the expression of deficient genes causing genetic disease, or to knock-out deleterious genetic sequences, either sexually inherited or introduced into the cells by infectious agents.

Since the emergence of the first programmable meganuclease reagents by the turn of the century (Smith et al. (2006) A combinatorial approach to create artificial homing endonucleases cleaving chosen sequences. Nucleic Acids Res. 34(22):e149), endonucleases reagents have rapidly evolved, offering improved specificity, safety and reliability. In particular, TALE-nucleases, which are fusions of a TALE binding domain with a cleavage catalytic domain (WO2011072246) have proven to be highly specific for therapeutic purposes (Leukaemia success heralds wave of gene-editing therapies (2015) Nature 527:146-147). TALE-nucleases are particularly specific when they are used by pairs under obligatory heterodimeric form, by using the dimeric cleavage domain of Fok-1. Left and right heterodimer members each recognizes a different nucleic sequences of about 14 to 20 bp, together spanning target sequences of 30 to 50 bp overall specificity.

More recently, further endonucleases reagents have been developed based on the components of the type II prokaryotic CRISPR (Clustered Regularly Interspaced Short palindromic Repeats) adaptive immune system of the bacteria S. *pyogenes.* This multicomponent system referred to as RNA-guided nuclease system (Gasiunas, Barrangou et al. 2012; Jinek, Chylinski et al. 2012), involves members of Cas9 or Cpf1 endonuclease families coupled with a guide RNA molecules that have the ability to drive said nuclease to some specific genome sequences (Zetsche et al. (2015). Cpf1 is a single RNA-guided endonuclease that provides immunity in bacteria and can be adapted for genome editing in mammalian cells. Cell 163:759-771). Such programmable RNA-guided endonucleases are easy to produce because the cleavage specificity is conferred by the sequence of the guide RNA, which can be cheaply adapted. Their specificity although stands on shorter sequences than TAL-nucleases of about 10 pb, which must be located near a particular motif (PAM) in the targeted genetic sequence. It has been reported a method for encapsulating a Cas9 endonuclease or a CRISPR guide RNA comprising producing the endonuclease or the guide sequence under RNA form, complexing said RNA with DLinDMA and PEG-S-DMG and forming particles of about 70 nm in diameter (WO2015/089462).

Various proofs of concept of the efficiency and safety of the above specific endonuclease reagents have been reported in human cells *in-vitro* or *ex-vivo*, but the delivery of the same reagents into the body has still to be very carefully considered due to the risk of off-site mutations inherent to such reagents. This risk increases when the reagents, which are mainly proteins, are being degraded in-vivo, altering their specificity and cleavage properties.

The primary challenge for gene therapy is thus to develop a method that delivers a therapeutic gene (e.g. transgene) or sequence specific reagent (e.g. nuclease) to selected cells where proper gene expression can be achieved. An ideal gene delivery method needs to meet 3 major criteria: (1) it should protect the transgene or reagent against degradation by nucleases in intercellular matrices, (2) it should bring the transgene or reagent across the plasma membrane and into the nucleus of target cells, and (3) it should have no detrimental effects (Gao, X. et al. (2007) Non-viral Gene Delivery: What we know and What is next. APPS Journal. 9(1) Article 9:92-104).

Viral vectors are able to mediate gene transfer with high efficiency and the possibility of long-term gene expression, may satisfy 2 out of 3 criteria. However, the acute immune response, immunogenicity, and insertion mutagenesis uncovered in gene therapy clinical trials have raised serious safety concerns about some commonly used viral vectors.

The inventors have explored safer means for *in-vivo* delivery and more particularly of endonucleases reagents, which could be used to target specific tissues into the human body especially delivery of sequence specific TAL-nucleases and Cas9/CRISPR into liver cells. They have determined that encapsulating nuclease reagent under RNA form in micelle structures of 50 to 100 nm was particularly appropriate to deliver the reagents into the nucleus of the cells by intravenous injection, in efficient amount and with limited off-site effects. This was primarily demonstrated by targeting cccDNA of HBV into liver cells, but this strategy has since been proven to be expandable to various type of cells by anchoring specific cell surface protein receptors or ligand into the micelle structures, as further detailed herein.

### Summary of the invention

The present disclosure which is not part of the invention is drawn to a method for encapsulating an endonuclease reagent, wherein said endonuclease reagent is prepared under RNA form and complexed with at least one biodegradable matrix comprising at least a core hydrophobic domain and a proximal polar domain to form particles of 50 to 100 nm diameter range, suitable for in-vivo injection.

The endonuclease reagent is generally a RNA molecule coding for a sequence-specific endonuclease reagent, such as a homing endonuclease, a zing finger nuclease, or a TALE-Nuclease, or a RNA guide optionally co-delivered with a RNA-guided endonuclease, such as cas9 or Cpf1.

Various types of biodegradable delivery capsules comprising RNA endonuclease reagents can be manufactured, depending on the structure of the biodegradable matrices involved and the monomers forming said core hydrophobic domain and polar domains.

Such biodegradable delivery capsules according to the invention are useful to deliver endonuclease reagent into the cells under RNA form, especially when co-delivery of different endonuclease reagents is sought, like for instance, messenger RNAs encoding right and left heterodimer TALE-nucleases.

Delivery specificity can be improved by linking a targeting domain to the proximal polar domain of said biodegradable matrix, such that the delivery capsules of the invention can bind surface antigens of different cell types. The delivery capsules are particularly suited for intravenous injection to target endogenous genetic sequences into cells.

The present application more particularly claims pharmaceutical compositions comprising the biodegradable delivery capsules of the invention into treatments involving endonuclease reagents. Such treatments may be part of a gene therapy, where specific genetic sequences have to be knocked-out or repaired, of an anti-infection therapy, by targeting the genome of infectious agents, or cancer therapy. The biodegradable delivery capsules of the present invention have proven to be particularly adapted to treat infectious agents that present a DNA intermediate in the liver cells, such as the cccDNA (covalently closed circular DNA) of Hepadnavirus, in particular HBV (Hepatitis B Virus), which are resistant forms of these viruses lodged into hepatocytes.

### Brief description of the figures and Tables:

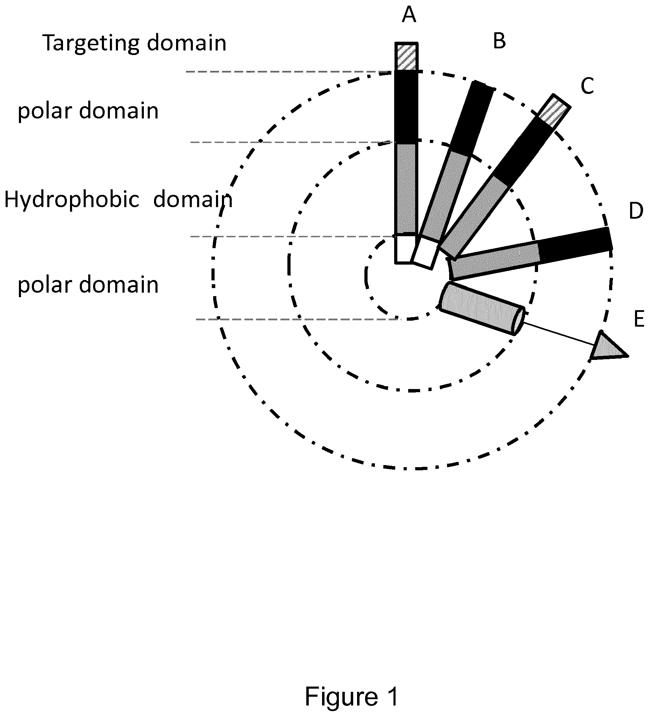

**Figure 1**:.Schematic representation of micelle-based system according to the invention: micelles can be obtained by mixing structures A, B, C, D (described here after) to form particles of 50 -100 nm diameter, by optionally incorporating protein E for extra targeting specificity. E is a protein or fusion protein containing a hydrophobic domain (for ex. derived from transmembrane protein) to anchor said protein within the micelle linked to a binding domain for endocytic receptor. The nuclease reagents are complexed to the micelles within the hydrophilic domains of A, B, C or D matrices.
   **A** and **B:** Structures comprising a distal hydrophilic domain conjugated to a hydrophobic domain, itself conjugated to a proximal hydrophilic domain complexed with the endonuclease reagents under RNA form. In A, said proximal hydrophilic domain is optionally linked to an external binding protein, such as N-acetylgalactosamine. These structures self-assemble under micelles that harbor a central hydrophilic pocket.
   **C** and **D:** Simpler structures consisting of a hydrophobic domain conjugated to the proximal hydrophilic domain, which is complexed with the endonuclease reagents under RNA form. In A, said proximal hydrophilic domain is optionally linked to an external binding protein, such as N-acetylgalactosamine.
**Figure 2****:** Schematic representation of the encapsulation of CRISPR based endonuclease reagents to perform gene editing *in vivo* according to the present invention. **A:** the guided endonuclease (ex: Cas9) is trapped into the inner hydrophilic core of the micelle, whereas the RNA guide is complexed into the polar domain of the matrix. **B:** the guided endonuclease (ex: Cas9) is first complexed with the RNA-guide to give a RNP (RiboNucleoProtein) that is complexed as such into the polar domain of the matrix.
**Figure 3****:** Schematic representation of the encapsulation heterodimeric TAL-nucleases endonuclease reagents to perform gene editing in vivo according to the present invention.
**Figure 4****:** Schematic representation of HBV genome cloned into cGPS HEK293 showing the position of the TALE-nucleases of the present invention.
**Figure 5****:** T7 endonuclease assays on TALEN set 1 (T002559 to T002564) chromatography gels and interpretation (Table 3)
**Figure 6****:** T7 endonuclease assays on TALEN set 2 (T001212 to T001215) chromatography gels and interpretation (Table 4)
**Table 1:** Exemplary list of target genes that can be modified by the gene editing method according to the invention and their associated diseases.
**Table 2:** : Engineered TAL-nucleases used in the *in-vivo* gene editing method to target HBV (cccDNA) and the genes encoding respectively APOC3, TTR, SMN2, IDOL, ANGPTL3, IDOL and PCSK9 (detailed target and polypeptide sequences are provided in Table 7).
**Table 3** (figure 5): Results of T7 for the HBV TALENs of set 1.
**Table 4** (figure 6): Results of T7 for the HBV TALENs of set 2.
**Table 5:** Quantitation of the PCR bands with Biorad Lab Image program obtained upon cleavage with the HBV TALENs of the present invention.
**Table 6:** Summary of the mice treatment schedule to target factor VII gene in the liver.
**Table 7:** TALE-nucleases engineered to target the HBV genome, APOC3, TTR, SMN2, IDOL, ANGPTL3 and PCSK9 genes, along with their polypeptide sequences et target polynucleotide sequences.

**Table 1: Exemplary list of target genes that can be modified in vivo by gene editing and the associated disease that can be treated according to the invention**

| **Target Gene** | **Symbol** | **NCBI gene ID** | **Disease** |
|---|---|---|---|
| proprotein convertase subtilisin/kexin type 9 | PCSK9 | 255738 | (Familial) hypercholesterolemia |
| apolipoprotein C-III | APOC3 | 345 | (Familial) hypertriglyceridemia/dyslipidemia |
| angiopoietin-like 3 | ANGPTL3 | 27329 | Combined hyperlipidemia/familial mixed hyperlipidemia |
| Inducible degrader of the LDLR, IDOL | MYLIP | 29116 | (Familial) hypercholesterolemia |
| transthyretin | TTR | 7276 | Transthyretin (TTR)-mediated amyloidosis (ATTR) |
| hydroxyacid oxidase (glycolate oxidase) 1 | HAO1 | 54363 | Primary hyperoxaluria type 1 (PH1) |
| serpin peptidase inhibitor, clade A | SERPINA1 | 5265 | Alpha 1-antitrypsin deficiency/COPD |
| transmembrane protease, serine 6 | TMPRSS6 | 164656 | Hemochromatosis and β-thalassemia |
| serpin peptidase inhibitor, clade C (antithrombin) | SERPINC1 | 462 | Haemophilia |
| solute carrier family 30 | SLC30A8 | 169026 | Diabetes mellitus type 2 |
| hepatitis B Virus | HBV | N.A. | Hepatitis - liver cancer |
| hungtingtin | HTT | 3064 | Huntington disease |
| myostatin | MSTN | 2660 | muscular degeneration |
| dystrophin | DMD | 13405 | Duchene muscular dystrophy |
| beta-2-microglobulin | B2M | 567 | graft |
| Cytomegalovirus | CMV | N.A. | Infectious disease |
| Herpes simplex Virus | HSV | N.A. | Infectious disease |
| Cystic Fibrosis Transmembrane Conductance Regulator | CFTR | 1080 | Cystic fibrosis |
| survival of motor neuron 2 | SMN2 | 6607 | Spinal muscular dystrophy |
| nicotinamide N-methyltransferase | NNMT | 4837 | obesity |
| chromosome 9 open reading frame 72 | C9ORF72 | 203228 | Amyotrophic lateral sclerosis |
| farnesyl-diphosphate farnesyltransferase 1 | FDFT1 | 2222 | (Familial) hypercholesterolemia |
| NPC1-like 1 | NPC1L1 | 29881 | (Familial) hypercholesterolemia |
| 3-hydroxy-3-methylglutaryl-CoA reductase | HMGCR | 3156 | (Familial) hypercholesterolemia |
| apolipoprotein B | APOB | 338 | (Familial) hypercholesterolemia |
| microsomal triglyceride transfer protein | MTTP | 4547 | (Familial) hypercholesterolemia |
| diacylglycerol O-acyltransferase 1 | DGAT1 | 8694 | (Familial) hypercholesterolemia |
| Hepatitis D virus | HDV | N.A. | infectious disease |
| Programmed death-ligand 1 | CD274 | 29126 | infectious disease |
| Fibroblast Growth Factor Receptor 4 | FGFR4 | 2264 | obesity |
| microRNA let-7 | let-7 | | cancer |
| microRNA miR-21 | miR-21 | | cancer |
| microRNA mir-26 | mir-26 | | cancer |
| microRNA mir-10 | mir-10 | | cancer |
| microRNA mir-34 | mir-34 | | cancer |
| microRNA mir-122 | mir-122 | | infectious disease |

**Table 2: Engineered TAL-nucleases used in the in-vivo gene editing method**

| **Target gene** | **Associated disease(s)** | **TALEN^{®} name** | **SEQ ID NO: #** | **Target sequence** |
|---|---|---|---|---|
| HBV | Hepatitis - liver cancer | T001212 | 1 | HBV1530_T01.L1 |
| | | T001212 | 2 | HBV1530_T01.R1 |
| | | T001213 | 3 | HBV1860_T01.L1 |
| | | T001213 | 4 | HBV1860_T01.R1 |
| | | T001214 | 5 | HBV2400_T01.L1 |
| | | T001214 | 6 | HBV2400_T01.R1 |
| | | T001215 | 7 | HBV180_T01.L1 |
| | | T001215 | 8 | HBV180_T01.R1 |
| | | T002559 | 9 | HBV-core-1.L1 |
| | | T002559 | 10 | HBV-core-1.R1 |
| | | T002560 | 11 | HBV-core-2.L1 |
| | | T002560 | 12 | HBV-core-2.R1 |
| | | T002561 | 13 | HBV-polym-1.L1 |
| | | T002561 | 14 | HBV-polym-1.R1 |
| | | T002562 | 15 | HBV-polym-2.L1 |
| | | T002562 | 16 | HBV-polym-2.R1 |
| | | T002563 | 17 | HBV-HBX-1.L1 |
| | | T002563 | 18 | HBV-HBX-1.R1 |
| | | T002564 | 19 | HBV-HBX-2.L1 |
| | | T002564 | 20 | HBV-HBX-2.R1 |
| APOC3 | (Familial) hypertriglyceridemia / dyslipidemia | T002657 | 25 | hAPOC3_Ex3A-L1 |
| | | T002657 | 26 | hAPOC3_Ex3A-R1 |
| | | T002658 | 27 | hAPOC3_Ex3B-L1 |
| | | T002658 | 28 | hAPOC3_Ex3B-R1 |
| | | T002671 | 29 | hAPOC3_Ex3_AN-R1 |
| | | T002671 | 30 | hAPOC3_Ex3A-L1 |
| TTR | Transthyretin (TTR)-mediated amyloidosis (ATTR) | T002659 | 31 | hTTR_Ex1A-L1 |
| | | T002659 | 32 | hTTR_Ex1A-R1 |
| | | T002660 | 33 | hTTR_Ex1B-L1 |
| | | T002660 | 34 | hTTR_Ex1B-R1 |
| SMN2 | Spinal muscular dystrophy | T002661 | 35 | hSMN2_3ssEx8A-L1 |
| | | T002661 | 36 | hSMN2_3ssEx8A-R1 |
| | | T002662 | 37 | hSMN2_3ssEx8B-L1 |
| | | T002662 | 38 | hSMN2_3ssEx8B-R1 |
| | | T002663 | 39 | hSMN2_ISS100A-L1 |
| | | T002663 | 40 | hSMN2_ISS100A-R1 |
| | | T002664 | 41 | hSMN2_ISS-N1A-L1 |
| | | T002664 | 42 | hSMN2_ISS-N1A-R1 |
| IDOL | | T002665 | 43 | hIDOL_Ex2A-L1 |
| | | T002665 | 44 | hIDOL_Ex2A-R1 |
| | | T002666 | 45 | hIDOL_Ex3A-L1 |
| | | T002666 | 46 | hIDOL_Ex3A-R1 |
| ANGPTL3 | Combined hyperlipidemia/famili al mixed hyperlipidemia | T002667 | 47 | hANGPTL3_Ex1A-L1 |
| | | T002667 | 48 | hANGPTL3_Ex1A-R1 |
| | | T002668 | 49 | hANGPTL3_Ex2A-L1 |
| | | T002668 | 50 | hANGPTL3_Ex2A-R1 |
| PCSK9 | (Familial) hypercholesterolemi a | T002669 | 51 | hPCSK9_Ex3A-L1 |
| | | T002669 | 52 | hPCSK9_Ex3A-R1 |
| | | T002670 | 53 | hPCSK9_Ex12A-L1 |
| | | T002670 | 54 | hPCSK9_Ex12A-R1 |
| | | T002672 | 55 | hPCSK9_Ex3_AN-L1 |
| | | T002672 | 56 | hPCSK9_Ex3A-R1 |
| | | T002673 | 57 | hPCSK9_Ex12A-L1 |
| | | T002673 | 58 | hPCSK9_Ex12A-R1 |

### Detailed description of the invention

Unless specifically defined herein, all technical and scientific terms used have the same meaning as commonly understood by a skilled artisan in the fields of gene therapy, biochemistry, genetics, and molecular biology.

All methods and materials similar or equivalent to those described herein can be used in the practice or testing of the present invention, with suitable methods and materials being described herein. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

In a general aspect, the present invention relates to a method for encapsulating a RNA molecule encoding a TALE-Nuclease, comprising the steps of:a) Engineering a RNA molecule encoding a TALE-Nuclease;b) Complexing the RNA molecule encoding said TALE-Nuclease with at least one biodegradable matrix comprising at least a core hydrophobic domain and a proximal polar domain to favor interactions with water molecules;c) Forming particles encapsulating said RNA molecule encoding said TALE-Nuclease of 50 to 100 nm diameter range; wherein said TALE-Nuclease binds a target sequence selected from SEQ ID NO: 59 to 78.

The particles are formed from by the association of macromolecular structures as shown in figure 1, which are detailed further on. These structures self-assemble due to their hydrophobic and hydrophilic domains upon rapid mixing by microfluidic mixing techniques, which permit millisecond mixing at the nanoliter scale with polydispersity indexes as low as, or lower than 0.02, as described by Song et al. (Microfluidic synthesis of nanomaterials (2008) Small 4:698-711). The particles may be formed by one or several types of those structures. Chimeric Proteins comprising a nonpolar or transmembrane domain and displaying a hydrophilic external affinity domain may be mixed with the other structural matrix structures to have these proteins anchored outwards the particles.

The elementary structures that build up the capsules by microfluidic mixing are preferably "biodegradable matrix", meaning that that they can be made of various materials that can be degraded or eliminated by action of the enzymes naturally present into the body, preferably into the human body.

By "diameter range" is meant that the diameter is not strictly uniform. It corresponds to a statistical measure (distribution of the diameter of a number of particles) centered on a major value. This value is generally comprised between 50 and 150 nm, and more generally preferably set between 50 and 100 nm, more preferably between 50 and 90 nm, and even more preferably between 60 and 80 nm.

By "endonuclease reagent" is meant a nucleic acid molecule that contributes to an endonuclease catalytic reaction in the target cell, itself or as a subunit of a complex, preferably leading to the cleavage of a nucleic acid sequence target. The "endonuclease reagents" are generally sequence-specific reagents, meaning that they can induce DNA cleavage in the cells at predetermined loci, referred to by extension as "gene targets", by specific recognition of a nucleic acid "target sequence". Said target sequence is usually selected to be rare or unique in the cell's genome, and more extensively in the human genome, as determined by using the available human genome databases and related common software.

"Rare-cutting endonucleases" are sequence-specific endonuclease reagents of choice, insofar as their recognition sequences generally range from10 to 50 successive base pairs, preferably from 12 to 30 bp, and more preferably from 14 to 20 bp.

According to a preferred aspect which is not part of the invention, said endonuclease reagent is a nucleic acid encoding an "engineered" or "programmable" rare-cutting endonuclease, such as a homing endonuclease as described for instance by Arnould S., et al. (WO2004067736), a zing finger nuclease as described, for instance, by Urnov F., et al. (Highly efficient endogenous human gene correction using designed zinc-finger nucleases (2005) Nature 435:646-651), a TALE-Nuclease as described, for instance, by Mussolino et al. (A novel TALE nuclease scaffold enables high genome editing activity in combination with low toxicity (2011) Nucl. Acids Res. 39(21):9283-9293), or a MegaTAL nuclease as described, for instance by Boissel et al. (MegaTALs: a rare-cleaving nuclease architecture for therapeutic genome engineering (2013) Nucleic Acids Research 42 (4):2591-2601).

According to the invention, the endonuclease reagent is preferentially under RNA form to allow transient endonuclease activity of said reagent into the target cell and make the entire capsule biodegradable *in-vivo.* Even more preferably, the endonuclease reagent is under the form of a mRNA for the expression of the rare cutting endonuclease into the cells. The endonuclease under mRNA form is preferably synthetized with a cap to enhance its stability according to techniques well known in the art, as described, for instance, by Kore A.L., et al. (Locked nucleic acid (LNA)-modified dinucleotide mRNA cap analogue: synthesis, enzymatic incorporation, and utilization (2009) J Am Chem Soc. 131(18):6364-5).

Due to their higher specificity, TALE-nuclease have proven to be particularly appropriate for therapeutic applications, especially under heterodimeric forms - i.e. working by pairs with a "right" monomer (also referred to as "5‴ or "forward") and 'left" monomer (also referred to as "3"" or "reverse") as reported for instance by Mussolino et al. (TALEN®facilitate targeted genome editing in human cells with high specificity and low cytotoxicity (2014) Nucl. Acids Res. 42(10): 6762-6773). However, before the invention, it was difficult to deliver in-vivo pairs of TALE-nuclease, even using viral vectors, especially because TAL-nuclease are large proteins having long genetic sequences. Delivery of these reactive proteins *in-vivo* was therefore remaining a considerable challenge before the present invention. This was all the more challenging that the proteins are active and specific when they are simultaneously delivered into the cell nucleus. Otherwise, the activity may be lost or the reagents may become less specific increasing the risk of off-site mutations.

The inventors have more particularly sought how to efficiently target cccDNA (covalently closed circular DNA), an intracellular viral replication intermediate of some viruses, which forms persistence reservoir and key obstacle for a cure of viral disease.

The cccDNA of HBV is at the origin of some common forms of chronic hepatitis B. Upon infection, cccDNA is generated as a plasmid-like episome in the host cell nucleus from the protein-linked relaxed circular (RC) DNA genome in incoming virions. It has a fundamental role as template for all viral RNAs, and in the production of new virions in the liver cells (Nassal et al., HBV cccDNA: viral persistence reservoir and key obstacle for a cure of chronic hepatitis B. (2015) Gut 64 (12):1972-1984).

Beyond the difficulty to deliver specific TALE-nucleases into the nucleus of liver cells, it must be recalled that HBV comprises different genotypes, at least 24 subtypes, displaying genome variability, which are reported to respond to treatment in different ways (Palumbo E., Hepatitis B genotypes and response to antiviral therapy: a review. (2007). American Journal of Therapeutics 14 (3): 306-9). As detailed in Example 1 herein, the inventors have designed specific TALE-nucleases that are able to target both the cccDNA of the main HBV subtypes, namely at least type A, B and C. The sequences of the successful TALE-nucleases used by the inventors to target HBV are listed in Table 7.

The present application claims any of the polypeptide or polynucleotide sequences having at least 80 % identity, preferably at least 90 %, more preferably 95%, and even more preferably 99% identity with any sequences referred to in Table 7. The present application claims the polynucleotides encoding said sequences, especially under RNA form.

It is more broadly provided a method, which is not part of the invention, for delivering *in-vivo* two endonuclease reagents under RNA form, preferably under mRNA, form to be expressed simultaneously into a cell.

Accordingly, it is provided a pair of heterodimeric TALE-nucleases, which is not part of the invention, preferably those targeting the cccDNA of HBV such as those referred in Table 2, which are encapsulated according to the method described herein to target liver cells - i.e.: into a biodegradable delivery capsule for gene targeting of a cell in vivo, characterized in that a endonuclease reagent under RNA form is complexed with at least one polar domain, which is linked to biodegradable conjugate(s) of hydrophobic monomers to form spherical particles as previously described.

According to another embodiment, the endonuclease reagent is a RNA-guide to be used in conjunction with a RNA guided endonuclease, such as Cas9 or Cpf1, as per, inter alia, the teaching by Doudna, J., and Chapentier, E., (The new frontier of genome engineering with CRISPR-Cas9 (2014) Science 346 (6213):1 077).

According to a preferred aspect the RNA guide is complexed with its associated RNA-guided endonuclease protein into the hydrophilic domain of the capsule. Alternatively the RNA-guided endonuclease protein can be retained within the hydrophilic core of the micelle structure (situation where the elementary structures have a second distal hydrophilic domain as illustrated in Figure 1 - structures A and B).

According to another embodiment, at least two different endonuclease reagents are encapsulated under RNA form into the particles, which means that, for instance, a RNA guide and mRNA encoding a RNA guided endonuclease can be both complexed with the distal hydrophilic domain of the matrix.

The present method provides that the core hydrophobic domain can be a biodegradable conjugate of hydrophobic monomers.

According to one aspect of the invention, said hydrophobic monomers conjugate comprise aminolipids, such as ionized cationic lipid 1,2-dilinoleyloxy-3-dimethylaminopropane (DLinDMA) as described elsewhere, for instance, by Hafez, I.M. et al. (On the mechanism whereby cationic lipids promote intracellular delivery of the polynucleic acids (2001) Gene Therapy 8:1188-1196). Said aminolipids can be advantageously mixed with PEG-lipids, since endogenous apolipoprotein E (Apo E) specifically targets these delivery systems to hepatocytes by Apo E-dependent, receptor mediated endocytosis. The core hydrophobic domain can also include structural lipids such as cholesterol and saturated phosphatidylcholine. Apo E mediated endocytosis is one of the mechanism by which capsules comprising hydrophobic domain with PEG-lipids more particularly target liver cells by mimicking intermediate-density lipoprotein (IDL).

According to an alternative aspect of the invention, said hydrophobic domain comprises a polymer, such as poly-N,N-di(C1-C6)alkyl-amino(C1-C6)alkyl-ethacrylate, poly-N,N-di(C1-C6)alkyl-amino(C1-C6)alkyl-methacrylate, or poly-N,N-di(C1-C6)alkylamino(C1-C6)alkyl-acrylate, or a combination thereof. Preferentially, said hydrophobic domain comprises monomers including at least (C2-C8)alkyl-ethacrylate, a (C2-C8)alkylmethacrylate, or a (C2-C8)alkyl-acrylate, which can be mixed with carboxylic acid monomers and tertiary amino monomers.

According to the present invention, a proximal polar "targeting domain" can be covalently linked to the core hydrophobic domain to specifically target desired cell type or tissue. Such targeting domain can be linked through usual peptide linker, such as Gly-rich linkers (GSₙ linkers) according to standard procedures known in the art. By "targeting domain" is meant any molecule that may be inserted or linked to the matrix providing more affinity of the capsule to a cell type, more generally to a specific cell surface marker.

Said targeting domain are mostly ligand or binding domains that are reported to have some affinity with cell surface receptors or antigens. Binding domains can be fusion proteins comprising ScFvs from antibodies generated against a cell surface antigen.

According to a preferred embodiment of the invention, the targeting domain recognizes a cell surface antigen from a LDL or VLDL receptor, which are abundantly present at the surface of liver cells, allowing easier internalization of the delivery capsule. According to another embodiment, the targeting domain has affinity with heparan sulfate proteoglycans.

According to another aspect of the invention N-acetylgalactosamine ligand is used as a targeting domain as a ligand of for asialoglycoprotein receptors (ASGP-r). Galactoside-containing cluster ligands, in particular glycopeptides containing N-acetyl-D-galactosamine (GaINAc) have high affinity to ASGP-r, which are found in abundance in mammalian parenchymal liver cells. Such ligands may be conjugated with the core hydrophobic domain to improve the efficiency of delivery to diseased liver cells as previously described by Wu Y.T., et al. (A new N-acetylgalactosamine containing peptide as a targeting vehicle for mammalian hepatocytes via asialoglycoprotein receptor endocytosis (2004) Curr Drug Deliv. Apr;1(2):119-27).

As per the method described above, the invention provides with biodegradable delivery capsule for performing gene targeting into a cell in-vivo. These delivery capsules, are, at least in part, characterized in that a RNA endonuclease reagent is complexed with at least one polar domain, which can be linked to biodegradable conjugate(s) of hydrophobic monomers, under the form of spherical particles of 50 to 100 nm diameter range. The structure of these delivery capsules allowed the inclusion of at least two RNA endonuclease reagents, such as TALE nucleases, which was surprising given the reduced size of the particles.

According to one aspect, said biodegradable matrix that is complexed with the RNA endonuclease reagents comprises at least two polar domains, in such a manner that the inner core particle is hydrophilic. The inner core particle may then encapsulate a further endonuclease reagent, in particular under polypeptide form, such as a RNA or DNA-guided endonuclease, for instance, a Cas9 or Cpf1 protein.

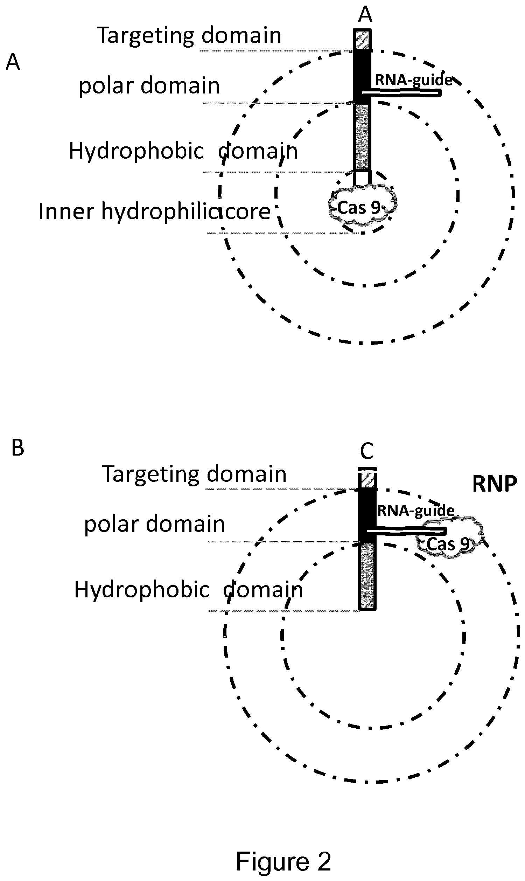

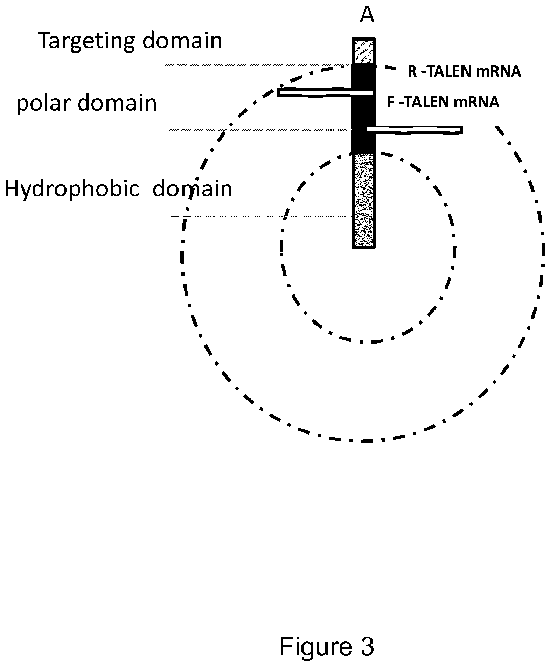

Various combinations of reagents can be included in the biodegradable delivery capsules of the present invention, as illustrated in figures 1 to 3.

The present invention is also drawn to a medicament or pharmaceutical compositions permitting the safe injection in-vivo of the above biodegradable delivery systems in view of editing a target gene. By "pharmaceutically injectable medium" is meant a suitable pharmaceutical carrier, which are well known in the art, such as phosphate buffered saline solutions, water, emulsions, such as oil/water emulsions, various types of wetting agents, sterile solutions, etc. Injections according to the present invention can be intracerebral, intramuscular, inside spinal chord or subcutaneous.

Preparations for intravenous administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like. In addition, the pharmaceutical composition of the present disclosure might comprise proteinaceous carriers, like, e.g., serum albumin or immunoglobulin, preferably of human origin.

The pharmaceutical compositions according to the present invention can have many different therapeutic indications. Examples of indications are those referred to in Table 1. Specifically, Table 1 lists particular target genes in connection with several diseases, which can be treated using the endonuclease delivery means according to the present invention. One particular aspect of this approach is the gene editing of these specific target genes in view of obtaining the treatment of their associated disease *in vivo.*

As a preferred embodiment is the therapeutic use of the endonuclease reagents to target in-vivo the genetic sequences expressing microRNAs involved into drug resistance in cancer treatment, especially after prolonged cycles of chemotherapy. Proposed targets in this respect are sequence encoding miRNA genes, especially those from let7, miR-21, mir-26, mir-10, mir-34 and/or mir-122 families. Drug resistance is a major problem in the treatment of cancer patients. Resistance can develop after prolonged cycles of chemotherapy or can be present intrinsically in the patient. There is an emerging role of microRNAs (miRNAs) in resistance to cancer treatments. miRNAs are small non-coding RNAs that are evolutionarily conserved and also involved as regulators of gene expression through the silencing of mRNA targets. They are involved in many different cancer types and a plethora of mechanisms have been postulated for the roles that miRNAs play in the development of drug resistance. Hence, miRNA-based gene therapy may provide a novel approach for the future of cancer therapy. This review focuses on an overview of recent findings on the role of miRNAs in the resistance to chemotherapy in different tumors.

According to a preferred embodiment of the invention, the biodegradable endonuclease delivery capsules are used against infection agents' genomes, in particular those agents that presents a DNA intermediate into the liver. By "DNA intermediate into the liver" is meant that the infectious agent has, even temporarily, at least one intermediate stage of its replication taking place into a hepatic cell under a DNA form.

HBV is such as infectious agent that presents a DNA intermediate as per its cccDNA, which forms a reservoir for the virus lodged into hepatic cells. Chronic hepatitis is mostly due to this cccDNA remaining in hepatic cells.

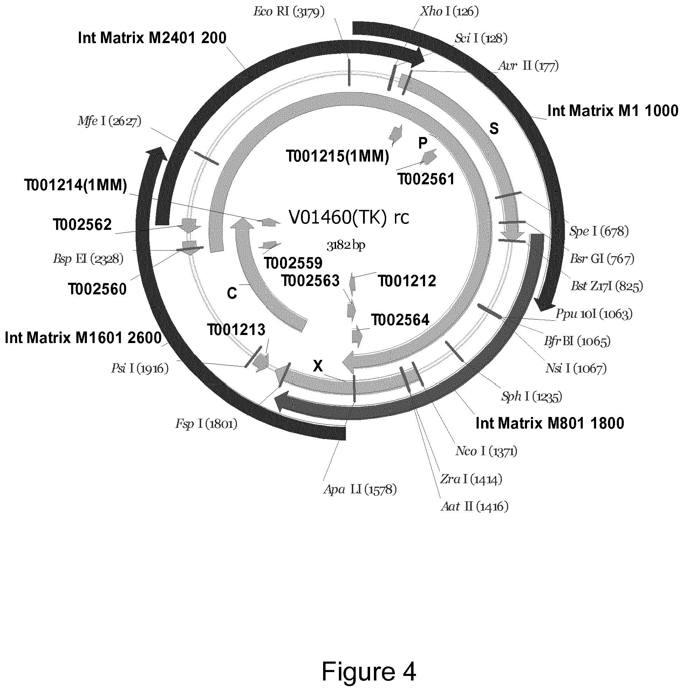

One aspect is to provide with new endonuclease reagents, especially TALE-nucleases (TALEN presented in Table 1) under RNA form for encapsulation into delivery particles for in-vivo targeting of HBV into liver cells. Preferred target sequences and TALEN monomers are those identified by the inventors in Example 1, through the cloning of large pieces of cccDNA into the genome of HEK293 cells (figure 4).

Also, it is provided TAL-nuclease monomers, which is not part of the invention, and which polypeptide sequence have at least 80% identity with any of SEQ ID NO.1 to 20, which are, useful for treating HBV related infections, and more particularly those having at least 80% identity with SEQ ID NO. 1, 2, 3, 4, 5, 6, 9, 10, 13, 14, 15, 16, 17 and 18 (TALEN 1212, 1213, 1214, 2559, 2561, 2562, and 2563 respectively displaying more than 40% activity in Example1).

With respect to antiviral treatments, and in particular those related to HBV, the endonuclease reagents of the present invention can be advantageously used in combination with other antiviral molecules, which do not target cccDNA, in particular those selected from: lamivudine (Epivir), entecavir (Baraclude), adefovir (Hepsera), tenofovir (Viread), Telbivudine (Tyzeka, Sebivo), Pegylated Interferon (Pegasys) or Interferon Alpha (Intron A).

A further aspect which is not part of the invention is a method for delivering an endonuclease reagent into a cell *in vivo,* comprising the step of:
- Producing a biodegradable delivery capsule as previously described; and
- Injecting, parenterally or enterally, preferably intravenously said capsule into the blood circulation of a patient;

More broadly, this aspect can be regarded as a method for gene editing a target gene into a cell in-vivo, comprising the steps of introducing into the blood stream of an animal a biodegradable delivery capsule comprising an endonuclease reagent under RNA form.

### Other definitions

- Amino acid residues in a polypeptide sequence are designated herein according to the one-letter code, in which, for example, Q means Gln or Glutamine residue, R means Arg or Arginine residue and D means Asp or Aspartic acid residue.
- Amino acid substitution means the replacement of one amino acid residue with another, for instance the replacement of an Arginine residue with a Glutamine residue in a peptide sequence is an amino acid substitution.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- "As used herein, "nucleic acid" or "polynucleotides" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Nucleic acids can be either single stranded or double stranded.
- The term "endonuclease" refers to any wild-type or variant enzyme capable of catalyzing the hydrolysis (cleavage) of bonds between nucleic acids within a DNA or RNA molecule, preferably a DNA molecule. Endonucleases do not cleave the DNA or RNA molecule irrespective of its sequence, but recognize and cleave the DNA or RNA molecule at specific polynucleotide sequences, further referred to as "target sequences" or "target sites". Endonucleases can be classified as rare-cutting endonucleases when having typically a polynucleotide recognition site greater than 10 base pairs (bp) in length, more preferably of 14-55 bp. Rare-cutting endonucleases significantly increase homologous recombination by inducing DNA double-strand breaks (DSBs) at a defined locus thereby allowing gene repair or gene insertion therapies (Pingoud, A. and G. H. Silva (2007). Precision genome surgery. Nat. Biotechnol. 25(7): 743-4.).
- by "DNA target", "DNA target sequence", "target DNA sequence", "nucleic acid target sequence", "target sequence" , or "processing site" is intended a polynucleotide sequence that can be targeted and processed by a rare-cutting endonuclease. These terms refer to a specific DNA location, preferably a genomic location in a cell, but also a portion of genetic material that can exist independently to the main body of genetic material such as plasmids, episomes, virus, transposons or in organelles such as mitochondria as non-limiting example. As non-limiting examples of RNA guided target sequences, are those genome sequences that can hybridize the guide RNA which directs the RNA guided endonuclease to a desired locus.
- By " delivery capsule" is intended any delivery mean which can be used to put into cell contact ( i.e "contacting") or deliver inside cells or subcellular compartments (i.e "introducing") the endonuclease reagents of the present invention. It includes, but is not limited to liposomal delivery vectors, viral delivery vectors, drug delivery vectors, chemical carriers, polymeric carriers, lipoplexes, polyplexes, dendrimers, microbubbles (ultrasound contrast agents), nanoparticles or emulsions.
- by "mutation" is intended the substitution, deletion, insertion of up to one, two, three, four, five, six, seven, eight, nine, ten, eleven, twelve, thirteen, fourteen, fifteen, twenty, twenty five, thirty, fourty, fifty, or more nucleotides/amino acids in a polynucleotide (cDNA, gene) or a polypeptide sequence. The mutation can affect the coding sequence of a gene or its regulatory sequence. It may also affect the structure of the genomic sequence or the structure/stability of the encoded mRNA.
- by "variant" is intended a catalytically active mutant of an endonuclease reagent.
- As used herein, the term "locus" is the specific physical location of a DNA sequence (e.g. of a gene) into a genome. The term "locus" can refer to the specific physical location of a rare-cutting endonuclease target sequence on a chromosome or on an infection agent's genome sequence. Such a locus can comprise a target sequence that is recognized and/or cleaved by a sequence-specific endonuclease. It is understood that the locus of interest can not only qualify a nucleic acid sequence that exists in the main body of genetic material (i.e. in a chromosome) of a cell but also a portion of genetic material that can exist independently to said main body of genetic material such as plasmids, episomes, virus, transposons or in organelles such as mitochondria as non-limiting examples.
- The term "cleavage" refers to the breakage of the covalent backbone of a polynucleotide. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. Double stranded DNA, RNA, or DNA/RNA hybrid cleavage can result in the production of either blunt ends or staggered ends.
- By "fusion protein" is intended the result of a well-known process in the art consisting in the joining of two or more genes which originally encode for separate proteins or part of them, the translation of said "fusion gene" resulting in a single polypeptide with functional properties derived from each of the original proteins.
- "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. For example, polypeptides having at least 70%, 85%, 90%, 95%, 98% or 99% identity to specific polypeptides described herein and preferably exhibiting substantially the same functions, as well as polynucleotide encoding such polypeptides, are contemplated.
- The term "subject" or "patient" as used herein includes all members of the animal kingdom including non-human primates and humans.
- The above written description of the invention provides a manner and process of making and using it such that any person skilled in this art is enabled to make and use the same, this enablement being provided in particular for the subject matter of the appended claims, which make up a part of the original description.

Where a numerical limit or range is stated herein, the endpoints are included. Also, all values and subranges within a numerical limit or range are specifically included as if explicitly written out.

Having generally described this invention, a further understanding can be obtained by reference to certain specific examples, which are provided herein for purposes of illustration only, and are not intended to be limiting unless otherwise specified.

### EXAMPLE 1

### Design of TAL-nucleases targeting HBV subtypes cccDNA

Hepatitis B virus (HBV) is a member of the Hepadnaviridae family of viruses. Infection with HBV can lead to cirrhosis and hepatocellular carcinoma. The genome of HBV is made of circular DNA, which is not fully double-stranded. One end of the full length strand is linked to the viral DNA polymerase. The genome is -3000 nucleotides long (for the full-length strand) and -2000 nucleotides long (for the short length-strand). The partially double-stranded DNA is rendered fully double-stranded by completion of the (+) sense strand and removal of a protein molecule from the (-) sense strand and a short sequence of RNA from the (+) sense strand. Non-coding bases are removed from the ends of the (-) sense strand and the ends are rejoined. There are four known genes encoded by the genome, called C, X, P, and S. The core protein is coded for by gene C (HBcAg), and its start codon is preceded by an upstream in-frame AUG start codon from which the pre-core protein is produced. HBeAg is produced by proteolytic processing of the pre-core protein. The DNA polymerase is encoded by gene P. Gene S is the gene that codes for the surface antigen (HBsAg). The function of the protein coded for by gene X is not fully understood but it is associated with the development of liver cancer. It stimulates genes that promote cell growth and inactivates growth regulating molecules.

The life cycle of hepatitis B virus is complex. Hepatitis B is one of a few known pararetroviruses: non-retroviruses that still use reverse transcription in their replication process. The virus gains entry into the cell by binding to NTCP on the surface and being endocytosed. Because the virus multiplies via RNA made by a host enzyme, the viral genomic DNA has to be transferred to the cell nucleus by cellular chaperones. The partially double stranded viral DNA is then made fully double stranded by viral polymerase and transformed into covalently closed circular DNA (cccDNA). This cccDNA serves as a template for transcription of four viral mRNAs by host RNA polymerase. The largest mRNA, (which is longer than the viral genome), is used to make the new copies of the genome and to make the capsid core protein and the viral DNA polymerase. These four viral transcripts undergo additional processing and go on to form progeny virions that are released from the cell or returned to the nucleus and re-cycled to produce more copies. The long mRNA is then transported back to the cytoplasm where the virion P protein (the DNA polymerase) synthesizes DNA via its reverse transcriptase activity.

The goal of the project was to generate TAL-nucleases able to cut HBV cccDNA and decrease its level in cell culture and in vivo. It should also cut the Relaxed Circular DNA (RC DNA), therefore acting of two different pools of virus. It could also cut integrated HBV partial genomes that are most often found in hepatocarcinomas.

There are no known inhibitors of the cccDNA. Some inhibitors are claimed to inhibit the formation of the cccDNA but none are targeting the pool of long lasting episomal reservoir of HBV in patients. The destruction of this pool could be a tool toward the cure of HBV.

HBV genome is difficult to express in cells line due to the complexity of its replication process and its toxicity. Also cells that produce HBV are difficult to transfect. We therefore decide to generate a stable cell line in 293 cells containing reference HBV genome (ayw) integrated at a known locus using a cGPS approach as described in WO2010046786, so that endonuclease reagent activity could be monitored in an easy and non -infectious manner.

HBV HEK 293 cells were very useful to select efficient TALE-nucleases targeting HBV. Cells M1-1000, M801-1800 and M1601-2006 were used to test TALE-nucleases: TALEN T002559-2564 (set 1) and TALEN T001212-1215 (set2). All of them were able to cut their target, with different level of efficiency. We were able to generate at least one very efficient TALEN per gene.

### PCR on transfected cGPS

The activity of the above TALENs was measured by transfecting plasmids in the above HBV 293 cGPS cells. 1.5 millions of each kind of HBV cGPS HEK 293 cells were plated in T25 the evening before transfection. 2 µg of each set of appropriate of plasmids (so 4 µg per TALEN, see table 1 and table 2) were transfected using Mirus TransIT-293 transfection reagent for 3 to 4 days. A control GFP was transfected in parallel (4 µg) for all cell lines and the efficiency of the transfection was monitored with a fluorescent microscope and estimated at -95%. Genomic DNA was then extracted using the Zymo Research quick-gDNA miniprep kit and PCR.

### T7 endonuclease assay on HBV cGPS cells tranfected with TALENs

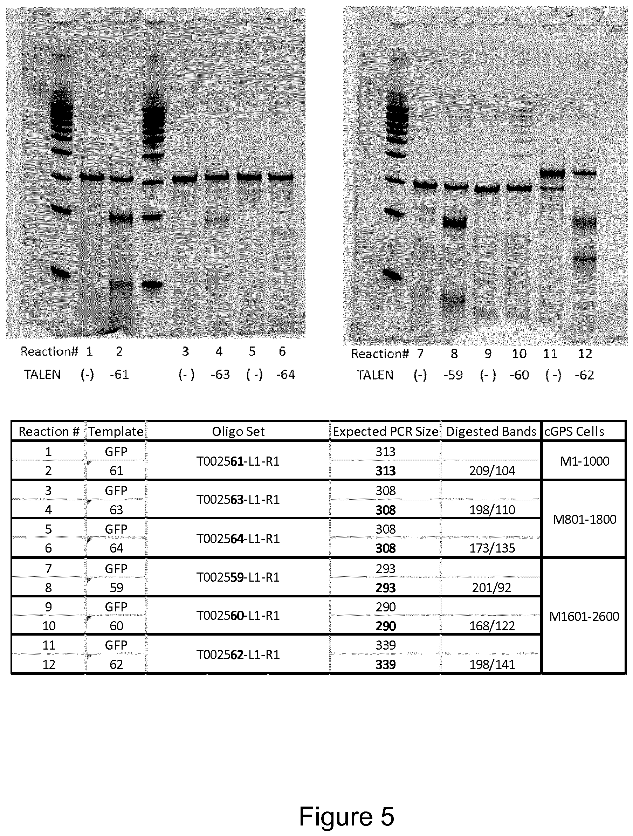

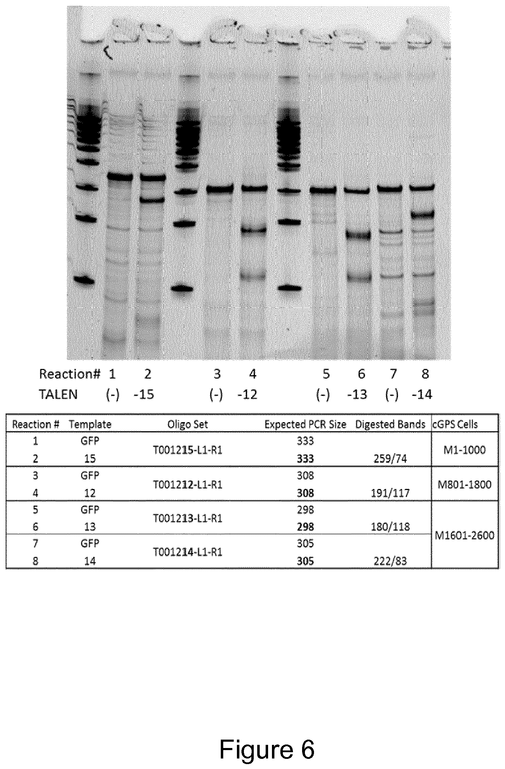

PCR products were annealed slowly, treated by T7 Endonuclease, run on a 10% polyacrylamide gel and stained with Sybergreen (T7 assay). One expects that the TALEN cut and NHEJ repair would result in the formation of a population of genomic DNA (and hence PCR product) that do not overlap at the surroundings of the TALEN cut site. After annealing those fragment would form a bulge that could be cleaved by the Endonuclease T7 and create fragments of expected sizes. Results are shown in figures 5 and 6. After T7 assay, digested fragments of PCR appeared at the expected size for all the TALEN tested. The PCR bands and cut the products presented in figures 5 and 6 were quantitated. Quantitation of digests with Biorad Image Lab program are presented in table 5. The efficiency of the cut was estimated by calculating the sum of the volume of the cut bands and dividing by the sum of the volume of total of the bands (cut and uncut). This approach does not take into account that the intensity of the bands is proportional to their size, and therefore this calculation is a rough estimate of the efficiency of the TALEN on the target.

All the TALEN tested had an estimated efficiency of more than 10%, while 6 were more than 40% active.

### Conclusion

HBV HEK 293 cells were very useful to select efficient TALENs targeting HBV. We were able to create at least one very efficient TALEN per gene. The activity of T002561 (which cuts S and P), T001212 (which cuts X and P), T002559 (which cuts C and P) was more than 40% based on T7 assay and are therefore eligible for being encapsulated for in-vivo targeting of liver cells.

**Table 5: Quantitation of the PCR bands with Biorad Lab Image program**

| **TALEN #** | **PCR bands** | **Volume** | | **SUM** | **Volume** | | **% Cut of Total** |
|---|---|---|---|---|---|---|---|
| **T002561** | PCR Uncut | 1624248 | | Total | 5073480 | | **68** |
| | PCR Cut 1 | 2180088 | | Cut | 3449232 | | |
| | PCR Cut 2 | 1269144 | | | | | |
| **T002563** | PCR Uncut | 2315160 | | Total | 3354624 | | **31** |
| | PCR Cut 1 | 684432 | | Cut | 1039464 | | |
| | PCR Cut 2 | 355032 | | | | | |
| **T002564** | PCR Uncut | 2654136 | | Total | 3024216 | | **12** |
| | PCR Cut 1 | 237312 | | Cut | 370080 | | |
| | PCR Cut 2 | 132768 | | | | | |
| **T002559** | PCR Uncut | 2056032 | | Total | 7750578 | | **73** |
| | PCR Cut 1 | 3415896 | | Cut | 5694546 | | |
| | PCR Cut 2 | 2278650 | | | | | |
| **T002560** | PCR Uncut | 2505030 | | Total | 3014946 | | **17** |
| | PCR Cut 1 | 308220 | | Cut | 509916 | | |
| | PCR Cut 2 | 201696 | | | | | |
| **T002562** | PCR Uncut | 1507440 | | Total | 5514894 | | **73** |
| | PCR Cut 1 | 2345508 | | Cut | 4007454 | | |
| | PCR Cut 2 | 1661946 | | | | | |
| **T001215** | PCR Uncut | 1675328 | | Total | 2451712 | | **32** |
| | PCR Cut 1 | 621312 | | Cut | 776384 | | |
| | PCR Cut 2 | 155072 | | | | | |
| **T001212** | PCR Uncut | 1453760 | | Total | 2613760 | | **44** |
| | PCR Cut 1 | 690688 | | Cut | 1160000 | | |
| | PCR Cut 2 | 469312 | | | | | |
| **T001213** | PCR Uncut | 939200 | | Total | 2262848 | | **58** |
| | PCR Cut 1 | 825408 | | Cut | 1323648 | | |
| | PCR Cut 2 | 498240 | | | | | |
| **T001214** | PCR Uncut | 975552 | | Total | 1789696 | | **45** |
| | PCR Cut 1 | 632000 | | Cut | 814144 | | |
| | PCR Cut 2 | 182144 | | | | | |

### EXAMPLE 2

### In-vivo targeting of Factor VII gene into the genome of liver cells

The liver is a key organ for most metabolic pathways and therefore numerous metabolic inherited diseases have their origin in this organ. It is an attractive target for *in vivo* gene transfer studies due to the accessibility of the hepatocytes via the blood stream. The liver is the largest organ in the body and a highly vascularized organ. It is the only organ in the body to have two circulation systems, the systemic with the hepatic artery that brings oxygenated blood directly from the heart and the portal vein that brings nutrients from the gut and supplies 70% of the blood flow to the liver. In addition, it has a system of ducts that transports toxins out of the liver via bile into the small intestine, which is of importance for liver gene therapy applications since it allows hepatocytes to excrete bile salts, copper, bilirubin, etc, which cause liver diseases. Candidate diseases for liver gene therapy include primary liver diseases in which hepatocytes are injured and genetic defects altering a specific function of the hepatocyte but causing extrahepatic manifestations.

As a proof of principle, the liver has been chosen as a target organ in view of inactivating *in-vivo* mouse factor VII . Mouse Factor VII is a secreted protein secreted by liver cells, which can be easily quantitated in the blood. Inactivation of the gene in mouse liver is expected to lead to a decrease of the secreted protein into the circulating blood as described elsewhere (Akin, A. et al. (2009) Development of Lipidoid-siRNA Formulations. Molecular Therapy 17 5: 872-879.)

TAL-nucleases were engineered at the DNA level using the golden gate cloning assembly method described by Weber E., et al. (Assembly of Designer TAL Effectors by Golden Gate Cloning (2011) PLoS ONE 6(5): e19722) and cloned into a mammalian expression vector under the control of a pEF1 alpha long promoter and tested in a human cell line by using a T7 experiment as described in Example 1.

Transcripts of mRNA encoding respectively forward (SEQ ID NO:21 or 23) and reverse (SEQ ID NO: 20 or 22) of the most efficient TALEN monomer pairs were produced using standard procedure (Ambion kit, Thermo Fisher Scientific). The capped transcripts were complexed with ionized cationic lipid 1,2-dilinoleyloxy-3-dimethylaminopropane (DLinDMA) and PEG- lipids to generate 80 nm range diameter range nanoparticles for systemic delivery to the liver.

Controls were PBS (negative control) and siRNA against Factor VII (positive control from Ambion, # 4457292 Thermofisher) as such siRNA have proven efficiency in previous studies.

The injection volume was 10 mL/kg (i.e for one mouse weighing 20g, 200 µL of dosing solution is administered). Injection were at 2mg/kg, of a 0.2mg/ml solution, so that 200µl were injected for a 20g mouse, intravenously (IV, *bolus*) into the caudal vein of mice.

The treatment starts on Day 0 (D₀) using sixteen (18) healthy female CD-1 mice.

The treatment schedule is as follows:
- The animals from group 1 received one single IV injection of Control Buffer (Q1Dx1),
- The animals from group 2 received one single IV injection of mRNA TALEN^{®} complexed into capsules at 2 mgRNA/kg (Q1Dx1),
- The animals from group 3 received one ingle IV injection of Positive siRNA control (siRNA FVII) at 2 mg/kg (Q1Dx1).

The treatment schedule is summarized in the table hereafter:

**Table 6: Mice treatment schedule**

| Group | Nb animals | Treatment | Dose (mg/kg/adm) | Adm. Route | Treatment schedule |
|---|---|---|---|---|---|
| 1 | 6 | Control Buffer | - | IV | Q1Dx1 |
| 2 | 6 | TALEN Complex | 2 mg/kg | IV | Q1Dx1 |
| 3 | 6 | Positive siRNA control | 2 mg/kg | IV | Q1Dx1 |

Blood was collected on D0 (just before IV treatment) and then on D3, D5, D10, D15, D20 and D25. All mice were terminated on D25. Approximately 150-200 µL of blood was collected at each time point, plasma was then flash-frozen in liquid nitrogen and stored at -80°C. Liver from each mouse was collected at the time of termination, flash-frozen in liquid nitrogen and stored at -80°C.

Factor VII activity in the collected plasma was measured using BIOPHEN FVII kit # 221304.

Gene modifications were measured in livers by extracting genomic DNA on powdered frozen liver using ZR Genomic DNA^{™} -Tissue MidiPrep from ZymoResearch # D3110, then performing a T7 assay and deep sequencing on the PCR product of the locus of interest.

Results show in figure 5, that a quickest effect is obtained with siRNA by D3 to D10. However by D15, siRNA starts to decline, whereas the inhibition induced by TALE-nucleases delivery remains stable up to D20. Together with the results of the deep sequencing (data not shown), it appears that the stable inhibition observed with the TALE-nucleases is due to mutations conferring permanent inactivation of a large number of factor VII gene copies in the liver cells.

**Table 7: TALE-nucleases engineered to target the HBV genome, APOC3, TTR, SMN2, IDOL, ANGPTL3 and PCSK9 genes, along with their polypeptide sequences et target polynucleotide sequences**

| TALEN Number | Gene target | RVD Motif | RVD polynucleotide target sequence | Polypeptide Sequence |
|---|---|---|---|---|
| T001212 | HBV1530_T01.L1 | HD-NG-NN-NG-NN-HD-HD-NG-NG-HD-NG-HD-NI-NG-HD-NG# | (SEQ ID NO:59) CTGTGCCTTCTC ATCT# | (SEQ ID NO:1) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPEQVVAIASHDGGKQALETVQRLLPVLCQAHGLTPQQVVAI ASNGGGKQALETVQRLLPVLCQAHGLTPQQVVAIASNNGGKQALETVQRLLPVLCQAHGLTPQQVV AIASNGGGKQALETVQRLLPVLCQAHGLTPQQVVAIASNNGGKQALETVQRLLPVLCQAHGLTPEQV VAIASHDGGKQALETVQRLLPVLCQAHGLTPEQVVAIASHDGGKQALETVQRLLPVLCQAHGLTPQQ VVAIASNGGGKQALETVQRLLPVLCQAHGLTPQQVVAIASNGGGKQALETVQRLLPVLCQAHGLTPE QVVAIASHDGGKQALETVQRLLPVLCQAHGLTPQQVVAIASNGGGKQALETVQRLLPVLCQAHGLTP EQVVAIASHDGGKQALETVQRLLPVLCQAHGLTPEQVVAIASNIGGKQALETVQALLPVLCQAHGLTP QQVVAIASNGGGKQALETVQRLLPVLCQAHGLTPEQVVAIASHDGGKQALETVQRLLPVLCQAHGLT PQQVVAIASNGGGRPALESIVAQLSRPDPALAALTNDHLVALACLGGRPALDAVKKGLGDPISRSQLV KSELEEKKSELRHKLKYVPHEYIELIEIARNSTQDRILEMKVMEFFMKVYGYRGKHLGGSRKPDGAIYT VGSPIDYGVIVDTKAYSGGYNLPIGQADEMQRYVEENQTRNKHINPNEWWKVYPSSVTEFKFLFVSG HFKGNYKAQLTRLNHITNCNGAVLSVEELLIGGEMIKAGTLTLEEVRRKFNNGEINFAAD |
| T001212 | HBV1530_T01.R1 | NN-HD-NI-NN-NI-NN-NN-NG-NN-NI-NI-NN-HD-NN-NI-NG# | (SEQ ID NO:60) GCAGAGGTGAA GCGAT# | (SEQ ID NO:2) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PEQVVAIASHDGGKQALETVQRLLPVLCQAHGLTPEQVVAIASNIGGKQALETVQALLPVLCQAHGLT PQQVVAIASNNGGKQALETVQRLLPVLCQAHGLTPEQVVAIASNIGGKQALETVQALLPVLCQAHGLT PQQVVAIASNNGGKQALETVQRLLPVLCQAHGLTPQQVVAIASNNGGKQALETVQRLLPVLCQAHGL TPQQVVAIASNGGGKQALETVQRLLPVLCQAHGLTPQQVVAIASNNGGKQALETVQRLLPVLCQAHG LTPEQVVAIASNIGGKQALETVQALLPVLCQAHGLTPEQVVAIASNIGGKQALETVQALLPVLCQAHGL TPQQVVAIASNNGGKQALETVQRLLPVLCQAHGLTPEQVVAIASHDGGKQALETVQRLLPVLCQAHG LTPQQVVAIASNNGGKQALETVQRLLPVLCQAHGLTPEQVVAIASNIGGKQALETVQALLPVLCQAHG LTPQQVVAIASNGGGRPALESIVAQLSRPDPALAALTNDHLVALACLGGRPALDAVKKGLGDPISRSQ LVKSELEEKKSELRHKLKYVPHEYIELIEIARNSTQDRILEMKVMEFFMKVYGYRGKHLGGSRKPDGAI YTVGSPIDYGVIVDTKAYSGGYNLPIGQADEMQRYVEENQTRNKHINPNEWWKVYPSSVTEFKFLFV SGHFKGNYKAQLTRLNHITNCNGAVLSVEELLIGGEMIKAGTLTLEEVRRKFNNGEINFAAD |
| T001213 | HBV1860_T01.L1 | NN-NG-NG-HD-NI-NI-NN-HD-HD-NG-HD-HD-NI-NI-NN-NG# | (SEQ ID NO:61) GTTCAAGCCTC CAAGT# | (SEQ ID NO:3) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNNGGKQALETVQRLLPVLCQAHGLTPQQVVAI ASNGGGKQALETVQRLLPVLCQAHGLTPQQVVAIASNGGGKQALETVQRLLPVLCQAHGLTPEQVV AIASHDGGKQALETVQRLLPVLCQAHGLTPEQVVAIASNIGGKQALETVQALLPVLCQAHGLTPEQVV AIASNIGGKQALETVQALLPVLCQAHGLTPQQVVAIASNNGGKQALETVQRLLPVLCQAHGLTPEQVV AIASHDGGKQALETVQRLLPVLCQAHGLTPEQVVAIASHDGGKQALETVQRLLPVLCQAHGLTPQQV VAIASNGGGKQALETVQRLLPVLCQAHGLTPEQVVAIASHDGGKQALETVQRLLPVLCQAHGLTPEQ VVAIASHDGGKQALETVQRLLPVLCQAHGLTPEQVVAIASNIGGKQALETVQALLPVLCQAHGLTPEQ VVAIASNIGGKQALETVQALLPVLCQAHGLTPQQVVAIASNNGGKQALETVQRLLPVLCQAHGLTPQQ VVAIASNGGGRPALESIVAQLSRPDPALAALTNDHLVALACLGGRPALDAVKKGLGDPISRSQLVKSEL EEKKSELRHKLKYVPHEYIELIEIARNSTQDRILEMKVMEFFMKVYGYRGKHLGGSRKPDGAIYTVGSP IDYGVIVDTKAYSGGYNLPIGQADEMQRYVEENQTRNKHINPNEWWKVYPSSVTEFKFLFVSGHFKG NYKAQLTRLNHITNCNGAVLSVEELLIGGEMIKAGTLTLEEVRRKFNNGEINFAAD |
| T001213 | HBV1860_T01.R1 | NN-NG-HD-HD-NI-NG-NN-HD-HD-HD-HD-NI-NI-NI-NN-NG# | (SEQ ID NO:62) GTCCATGCCCC AAAGT# | (SEQ ID NO:4) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PQQVVAIASNGGGKQALETVQRLLPVLCQAHGLTPEQVVAIASHDGGKQALETVQRLLPVLCQAHGL TPEQVVAIASHDGGKQALETVQRLLPVLCQAHGLTPEQVVAIASNIGGKQALETVQALLPVLCQAHGL TPQQVVAIASNGGGKQALETVQRLLPVLCQAHGLTPQQVVAIASNNGGKQALETVQRLLPVLCQAHG LTPEQVVAIASHDGGKQALETVQRLLPVLCQAHGLTPEQVVAIASHDGGKQALETVQRLLPVLCQAH GLTPEQVVAIASHDGGKQALETVQRLLPVLCQAHGLTPEQVVAIASHDGGKQALETVQRLLPVLCQA HGLTPEQVVAIASNIGGKQALETVQALLPVLCQAHGLTPEQVVAIASNIGGKQALETVQALLPVLCQAH GLTPEQVVAIASNIGGKQALETVQALLPVLCQAHGLTPQQVVAIASNNGGKQALETVQRLLPVLCQAH GLTPQQVVAIASNGGGRPALESIVAQLSRPDPALAALTNDHLVALACLGGRPALDAVKKGLGDPISRS QLVKSELEEKKSELRHKLKYVPHEYIELIEIARNSTQDRILEMKVMEFFMKVYGYRGKHLGGSRKPDG AIYTVGSPIDYGVIVDTKAYSGGYNLPIGQADEMQRYVEENQTRNKHINPNEWWKVYPSSVTEFKFLF VSGHFKGNYKAQLTRLNHITNCNGAVLSVEELLIGGEMIKAGTLTLEEVRRKFNNGEINFAAD |
| T001214 | HBV2400_T01.L1 | HD-NN-HD-NI-NN-NI-NI-NN-NI-NG-HD-NG-HD-NI-NI-NG# | (SEQ ID NO:63) CGCAGAAGATC TCAAT# | (SEQ ID NO:5) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPEQVVAIASHDGGKQALETVQRLLPVLCQAHGLTPQQVVAI ASNNGGKQALETVQRLLPVLCQAHGLTPEQVVAIASHDGGKQALETVQRLLPVLCQAHGLTPEQVVA IASNIGGKQALETVQALLPVLCQAHGLTPQQVVAIASNNGGKQALETVQRLLPVLCQAHGLTPEQVVAI ASNIGGKQALETVQALLPVLCQAHGLTPEQVVAIASNIGGKQALETVQALLPVLCQAHGLTPQQVVAIA SNNGGKQALETVQRLLPVLCQAHGLTPEQVVAIASNIGGKQALETVQALLPVLCQAHGLTPQQVVAIA SNGGGKQALETVQRLLPVLCQAHGLTPEQVVAIASHDGGKQALETVQRLLPVLCQAHGLTPQQVVAI ASNGGGKQALETVQRLLPVLCQAHGLTPEQVVAIASHDGGKQALETVQRLLPVLCQAHGLTPEQVVA IASNIGGKQALETVQALLPVLCQAHGLTPEQVVAIASNIGGKQALETVQALLPVLCQAHGLTPQQVVAI ASNGGGRPALESIVAQLSRPDPALAALTNDHLVALACLGGRPALDAVKKGLGDPISRSQLVKSELEEK KSELRHKLKYVPHEYIELIEIARNSTQDRILEMKVMEFFMKVYGYRGKHLGGSRKPDGAIYTVGSPIDY GVIVDTKAYSGGYNLPIGQADEMQRYVEENQTRNKHINPNEWWKVYPSSVTEFKFLFVSGHFKGNY KAQLTRLNHITNCNGAVLSVEELLIGGEMIKAGTLTLEEVRRKFNNGEINFAAD |
| T001214 | HBV2400_T01.R1 | HD-HD-NI-NI-NN-NN-NN-NI-NG-NI-HD-NG-NI-NI-HD-NG# | (SEQ ID NO:64) CCAAGGGATAC TAACT# | (SEQ ID NO:6) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPEQVVAIASHDGGKQALETVQRLLPVLCQAHGLT PEQVVAIASHDGGKQALETVQRLLPVLCQAHGLTPEQVVAIASNIGGKQALETVQALLPVLCQAHGLT PEQVVAIASNIGGKQALETVQALLPVLCQAHGLTPQQVVAIASNNGGKQALETVQRLLPVLCQAHGLT PQQVVAIASNNGGKQALETVQRLLPVLCQAHGLTPQQVVAIASNNGGKQALETVQRLLPVLCQAHGL TPEQVVAIASNIGGKQALETVQALLPVLCQAHGLTPQQVVAIASNGGGKQALETVQRLLPVLCQAHGL TPEQVVAIASNIGGKQALETVQALLPVLCQAHGLTPEQVVAIASHDGGKQALETVQRLLPVLCQAHGL TPQQVVAIASNGGGKQALETVQRLLPVLCQAHGLTPEQVVAIASNIGGKQALETVQALLPVLCQAHGL TPEQVVAIASNIGGKQALETVQALLPVLCQAHGLTPEQVVAIASHDGGKQALETVQRLLPVLCQAHGL TPQQVVAIASNGGGRPALESIVAQLSRPDPALAALTNDHLVALACLGGRPALDAVKKGLGDPISRSQL VKSELEEKKSELRHKLKYVPHEYIELIEIARNSTQDRILEMKVMEFFMKVYGYRGKHLGGSRKPDGAIY TVGSPIDYGVIVDTKAYSGGYNLPIGQADEMQRYVEENQTRNKHINPNEWWKVYPSSVTEFKFLFVS GHFKGNYKAQLTRLNHITNCNGAVLSVEELLIGGEMIKAGTLTLEEVRRKFNNGEINFAAD |
| T001215 | HBV180_T01.L1 | NN-NG-NG-NI-HD-NI-NN-NN-HD-NN-NN-NN-NN-NG-NG-NG# | (SEQ ID NO:65) GTTACAGGCGG GGTTT# | (SEQ ID NO:7) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNNGGKQALETVQRLLPVLCQAHGLTPQQVVAI ASNGGGKQALETVQRLLPVLCQAHGLTPQQVVAIASNGGGKQALETVQRLLPVLCQAHGLTPEQVV AIASNIGGKQALETVQALLPVLCQAHGLTPEQVVAIASHDGGKQALETVQRLLPVLCQAHGLTPEQVV AIASNIGGKQALETVQALLPVLCQAHGLTPQQVVAIASNNGGKQALETVQRLLPVLCQAHGLTPQQVV AIASNNGGKQALETVQRLLPVLCQAHGLTPEQVVAIASHDGGKQALETVQRLLPVLCQAHGLTPQQV VAIASNNGGKQALETVQRLLPVLCQAHGLTPQQVVAIASNNGGKQALETVQRLLPVLCQAHGLTPQQ VVAIASNNGGKQALETVQRLLPVLCQAHGLTPQQVVAIASNNGGKQALETVQRLLPVLCQAHGLTPQ QVVAIASNGGGKQALETVQRLLPVLCQAHGLTPQQVVAIASNGGGKQALETVQRLLPVLCQAHGLTP QQVVAIASNGGGRPALESIVAQLSRPDPALAALTNDHLVALACLGGRPALDAVKKGLGDPISRSQLVK SELEEKKSELRHKLKYVPHEYIELIEIARNSTQDRILEMKVMEFFMKVYGYRGKHLGGSRKPDGAIYTV GSPIDYGVIVDTKAYSGGYNLPIGQADEMQRYVEENQTRNKHINPNEWWKVYPSSVTEFKFLFVSGH FKGNYKAQLTRLNHITNCNGAVLSVEELLIGGEMIKAGTLTLEEVRRKFNNGEINFAAD |
| T001215 | HBV180_T01.R1 | NN-NG-NN-NN-NG-NI-NG-NG-NN-NG-NN-NI-NN-NN-NI-NG# | (SEQ ID NO:66) GTGGTATTGTG AGGAT# | (SEQ ID NO:8) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PQQVVAIASNGGGKQALETVQRLLPVLCQAHGLTPQQVVAIASNNGGKQALETVQRLLPVLCQAHGL TPQQVVAIASNNGGKQALETVQRLLPVLCQAHGLTPQQVVAIASNGGGKQALETVQRLLPVLCQAHG LTPEQVVAIASNIGGKQALETVQALLPVLCQAHGLTPQQVVAIASNGGGKQALETVQRLLPVLCQAHG LTPQQVVAIASNGGGKQALETVQRLLPVLCQAHGLTPQQVVAIASNNGGKQALETVQRLLPVLCQAH GLTPQQVVAIASNGGGKQALETVQRLLPVLCQAHGLTPQQVVAIASNNGGKQALETVQRLLPVLCQA HGLTPEQVVAIASNIGGKQALETVQALLPVLCQAHGLTPQQVVAIASNNGGKQALETVQRLLPVLCQA HGLTPQQVVAIASNNGGKQALETVQRLLPVLCQAHGLTPEQVVAIASNIGGKQALETVQALLPVLCQA HGLTPQQVVAIASNGGGRPALESIVAQLSRPDPALAALTNDHLVALACLGGRPALDAVKKGLGDPISR SQLVKSELEEKKSELRHKLKYVPHEYIELIEIARNSTQDRILEMKVMEFFMKVYGYRGKHLGGSRKPD GAIYTVGSPIDYGVIVDTKAYSGGYNLPIGQADEMQRYVEENQTRNKHINPNEWWKVYPSSVTEFKFL FVSGHFKGNYKAQLTRLNHITNCNGAVLSVEELLIGGEMIKAGTLTLEEVRRKFNNGEINFAAD |
| T002559 | HBV-core-1.L1 | NN-NG-NN-NN-NI-NG-NG-HD-NN-HD-NI-HD-NG-HD-HD-NG# | (SEQ ID NO:67) GTGGATTCGCA CTCCT# | (SEQ ID NO:9) MGDPKKKRKVIDYPYDVPDYAIDIADPIRSRTPSPARELLPGPQPDGVQPTADRGVSPPAGGPLDGLP ARRTMSRTRLPSPPAPSPAFSAGSFSDLLRQFDPSLFNTSLFDSLPPFGAHHTEAATGEWDEVQSGL RAADAPPPTMRVAVTAARPPRAKPAPRRRAAQPSDASPAAQVDLRTLGYSQQQQEKIKPKVRSTVA QHHEALVGHGFTHAHIVALSQHPAALGTVAVKYQDMIAALPEATHEAIVGVGKQWSGARALEALLTVA GELRGPPLQLDTGQLLKIAKRGGVTAVEAVHAWRNALTGAPLNLTPQQVVAIASNNGGKQALETVQR LLPVLCQAHGLTPQQVVAIASNGGGKQALETVQRLLPVLCQAHGLTPQQVVAIASNNGGKQALETVQ RLLPVLCQAHGLTPQQVVAIASNNGGKQALETVQRLLPVLCQAHGLTPEQVVAIASNIGGKQALETVQ ALLPVLCQAHGLTPQQVVAIASNGGGKQALETVQRLLPVLCQAHGLTPQQVVAIASNGGGKQALETV QRLLPVLCQAHGLTPEQVVAIASHDGGKQALETVQRLLPVLCQAHGLTPQQVVAIASNNGGKQALET VQRLLPVLCQAHGLTPEQVVAIASHDGGKQALETVQRLLPVLCQAHGLTPEQVVAIASNIGGKQALET VQALLPVLCQAHGLTPEQVVAIASHDGGKQALETVQRLLPVLCQAHGLTPQQVVAIASNGGGKQALE TVQRLLPVLCQAHGLTPEQVVAIASHDGGKQALETVQRLLPVLCQAHGLTPEQVVAIASHDGGKQAL ETVQRLLPVLCQAHGLTPQQVVAIASNGGGRPALESIVAQLSRPDPSGSGSGGDPISRSQLVKSELEE KKSELRHKLKYVPHEYIELIEIARNSTQDRILEMKVMEFFMKVYGYRGKHLGGSRKPDGAIYTVGSPID YGVIVDTKAYSGGYNLPIGQADEMQRYVEENQTRNKHINPNEWWKVYPSSVTEFKFLFVSGHFKGN YKAQLTRLNHITNCNGAVLSVEELLIGGEMIKAGTLTLEEVRRKFNNGEINFAAD |
| T002559 | HBV-core-1.R1 | NI-NN-NN-NN-NN-HD-NI-NG-NG-NG-NN-NN-NG-NN-NN-NG# | (SEQ ID NO:68) AGGGGCATTTG GTGGT# | (SEQ ID NO:10) MGDPKKKRKVIDKETAAAKFERQHMDSIDIADPIRSRTPSPARELLPGPQPDGVQPTADRGVSPPAG GPLDGLPARRTMSRTRLPSPPAPSPAFSAGSFSDLLRQFDPSLFNTSLFDSLPPFGAHHTEAATGEW DEVQSGLRAADAPPPTMRVAVTAARPPRAKPAPRRRAAQPSDASPAAQVDLRTLGYSQQQQEKIKP KVRSTVAQHHEALVGHGFTHAHIVALSQHPAALGTVAVKYQDMIAALPEATHEAIVGVGKQWSGARA LEALLTVAGELRGPPLQLDTGQLLKIAKRGGVTAVEAVHAWRNALTGAPLNLTPEQVVAIASNIGGKQ ALETVQALLPVLCQAHGLTPQQVVAIASNNGGKQALETVQRLLPVLCQAHGLTPQQVVAIASNNGGK QALETVQRLLPVLCQAHGLTPQQVVAIASNNGGKQALETVQRLLPVLCQAHGLTPQQVVAIASNNGG KQALETVQRLLPVLCQAHGLTPEQVVAIASHDGGKQALETVQRLLPVLCQAHGLTPEQVVAIASNIGG KQALETVQALLPVLCQAHGLTPQQVVAIASNGGGKQALETVQRLLPVLCQAHGLTPQQVVAIASNGG GKQALETVQRLLPVLCQAHGLTPQQVVAIASNGGGKQALETVQRLLPVLCQAHGLTPQQVVAIASNN GGKQALETVQRLLPVLCQAHGLTPQQVVAIASNNGGKQALETVQRLLPVLCQAHGLTPQQVVAIASN GGGKQALETVQRLLPVLCQAHGLTPQQVVAIASNNGGKQALETVQRLLPVLCQAHGLTPQQVVAIAS NNGGKQALETVQRLLPVLCQAHGLTPQQVVAIASNGGGRPALESIVAQLSRPDPSGSGSGGDPISRS QLVKSELEEKKSELRHKLKYVPHEYIELIEIARNSTQDRILEMKVMEFFMKVYGYRGKHLGGSRKPDG AIYTVGSPIDYGVIVDTKAYSGGYNLPIGQADEMQRYVEENQTRNKHINPNEWWKVYPSSVTEFKFLF VSGHFKGNYKAQLTRLNHITNCNGAVLSVEELLIGGEMIKAGTLTLEEVRRKFNNGEINFAAD |
| T002560 | HBV-core-2.L1 | NN-HD-HD-HD-HD-NG-NI-NG-HD-NG-NG-NI-NG-HD-NI-NG# | (SEQ ID NO:69) GCCCCTATCTTA TCAT# | (SEQ ID NO:11) MGDPKKKRKVIDYPYDVPDYAIDIADPIRSRTPSPARELLPGPQPDGVQPTADRGVSPPAGGPLDGLP ARRTMSRTRLPSPPAPSPAFSAGSFSDLLRQFDPSLFNTSLFDSLPPFGAHHTEAATGEWDEVQSGL RAADAPPPTMRVAVTAARPPRAKPAPRRRAAQPSDASPAAQVDLRTLGYSQQQQEKIKPKVRSTVA QHHEALVGHGFTHAHIVALSQHPAALGTVAVKYQDMIAALPEATHEAIVGVGKQWSGARALEALLTVA GELRGPPLQLDTGQLLKIAKRGGVTAVEAVHAWRNALTGAPLNLTPQQVVAIASNNGGKQALETVQR LLPVLCQAHGLTPEQVVAIASHDGGKQALETVQRLLPVLCQAHGLTPEQVVAIASHDGGKQALETVQ RLLPVLCQAHGLTPEQVVAIASHDGGKQALETVQRLLPVLCQAHGLTPEQVVAIASHDGGKQALETV QRLLPVLCQAHGLTPQQVVAIASNGGGKQALETVQRLLPVLCQAHGLTPEQVVAIASNIGGKQALETV QALLPVLCQAHGLTPQQVVAIASNGGGKQALETVQRLLPVLCQAHGLTPEQVVAIASHDGGKQALET VQRLLPVLCQAHGLTPQQVVAIASNGGGKQALETVQRLLPVLCQAHGLTPQQVVAIASNGGGKQALE TVQRLLPVLCQAHGLTPEQVVAIASNIGGKQALETVQALLPVLCQAHGLTPQQVVAIASNGGGKQALE TVQRLLPVLCQAHGLTPEQVVAIASHDGGKQALETVQRLLPVLCQAHGLTPEQVVAIASNIGGKQALE TVQALLPVLCQAHGLTPQQVVAIASNGGGRPALESIVAQLSRPDPSGSGSGGDPISRSQLVKSELEEK KSELRHKLKYVPHEYIELIEIARNSTQDRILEMKVMEFFMKVYGYRGKHLGGSRKPDGAIYTVGSPIDY GVIVDTKAYSGGYNLPIGQADEMQRYVEENQTRNKHINPNEWWKVYPSSVTEFKFLFVSGHFKGNY KAQLTRLNHITNCNGAVLSVEELLIGGEMIKAGTLTLEEVRRKFNNGEINFAAD |
| T002560 | HBV-core-2.R1 | HD-NN-NG-HD-NG-NI-NI-HD-NI-NI-HD-NI-NN-NG-NI-NG# | (SEQ ID NO:70) CGTCTAACAACA GTAT# | (SEQ ID NO:12) MGDPKKKRKVIDKETAAAKFERQHMDSIDIADPIRSRTPSPARELLPGPQPDGVQPTADRGVSPPAG GPLDGLPARRTMSRTRLPSPPAPSPAFSAGSFSDLLRQFDPSLFNTSLFDSLPPFGAHHTEAATGEW DEVQSGLRAADAPPPTMRVAVTAARPPRAKPAPRRRAAQPSDASPAAQVDLRTLGYSQQQQEKIKP KVRSTVAQHHEALVGHGFTHAHIVALSQHPAALGTVAVKYQDMIAALPEATHEAIVGVGKQWSGARA LEALLTVAGELRGPPLQLDTGQLLKIAKRGGVTAVEAVHAWRNALTGAPLNLTPEQVVAIASHDGGKQ ALETVQRLLPVLCQAHGLTPQQVVAIASNNGGKQALETVQRLLPVLCQAHGLTPQQVVAIASNGGGK QALETVQRLLPVLCQAHGLTPEQVVAIASHDGGKQALETVQRLLPVLCQAHGLTPQQVVAIASNGGG KQALETVQRLLPVLCQAHGLTPEQVVAIASNIGGKQALETVQALLPVLCQAHGLTPEQVVAIASNIGGK QALETVQALLPVLCQAHGLTPEQVVAIASHDGGKQALETVQRLLPVLCQAHGLTPEQVVAIASNIGGK QALETVQALLPVLCQAHGLTPEQVVAIASNIGGKQALETVQALLPVLCQAHGLTPEQVVAIASHDGGK QALETVQRLLPVLCQAHGLTPEQVVAIASNIGGKQALETVQALLPVLCQAHGLTPQQVVAIASNNGGK QALETVQRLLPVLCQAHGLTPQQVVAIASNGGGKQALETVQRLLPVLCQAHGLTPEQVVAIASNIGGK QALETVQALLPVLCQAHGLTPQQVVAIASNGGGRPALESIVAQLSRPDPSGSGSGGDPISRSQLVKS ELEEKKSELRHKLKYVPHEYIELIEIARNSTQDRILEMKVMEFFMKVYGYRGKHLGGSRKPDGAIYTVG SPIDYGVIVDTKAYSGGYNLPIGQADEMQRYVEENQTRNKHINPNEWWKVYPSSVTEFKFLFVSGHF KGNYKAQLTRLNHITNCNGAVLSVEELLIGGEMIKAGTLTLEEVRRKFNNGEINFAAD |
| T002561 | HBV-polym-1.L1 | NN-NG-HD-NG-NN-HD-NN-NN-HD-NN-NG-NG-NG-NG-NI-NG# | (SEQ ID NO:71) GTCTGCGGCGT TTTAT# | (SEQ ID NO:13) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNNGGKQALETVQRLLPVLCQAHGLTPQQVVAI ASNGGGKQALETVQRLLPVLCQAHGLTPEQVVAIASHDGGKQALETVQRLLPVLCQAHGLTPQQVVA IASNGGGKQALETVQRLLPVLCQAHGLTPQQVVAIASNNGGKQALETVQRLLPVLCQAHGLTPEQVV AIASHDGGKQALETVQRLLPVLCQAHGLTPQQVVAIASNNGGKQALETVQRLLPVLCQAHGLTPQQV VAIASNNGGKQALETVQRLLPVLCQAHGLTPEQVVAIASHDGGKQALETVQRLLPVLCQAHGLTPQQ VVAIASNNGGKQALETVQRLLPVLCQAHGLTPQQVVAIASNGGGKQALETVQRLLPVLCQAHGLTPQ QVVAIASNGGGKQALETVQRLLPVLCQAHGLTPQQVVAIASNGGGKQALETVQRLLPVLCQAHGLTP QQVVAIASNGGGKQALETVQRLLPVLCQAHGLTPEQVVAIASNIGGKQALETVQALLPVLCQAHGLTP QQVVAIASNGGGRPALESIVAQLSRPDPALAALTNDHLVALACLGGRPALDAVKKGLGDPISRSQLVK SELEEKKSELRHKLKYVPHEYIELIEIARNSTQDRILEMKVMEFFMKVYGYRGKHLGGSRKPDGAIYTV GSPIDYGVIVDTKAYSGGYNLPIGQADEMQRYVEENQTRNKHINPNEWWKVYPSSVTEFKFLFVSGH FKGNYKAQLTRLNHITNCNGAVLSVEELLIGGEMIKAGTLTLEEVRRKFNNGEINFAAD |
| T002561 | HBV-polym-1.R1 | NN-NI-NN-NN-HD-NI-NG-NI-NN-HD-NI-NN-HD-NI-NN-NG# | (SEQ ID NO:72) GAGGCATAGCA GCAGT# | (SEQ ID NO:14) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PEQVVAIASNIGGKQALETVQALLPVLCQAHGLTPQQVVAIASNNGGKQALETVQRLLPVLCQAHGLT PQQVVAIASNNGGKQALETVQRLLPVLCQAHGLTPEQVVAIASHDGGKQALETVQRLLPVLCQAHGL TPEQVVAIASNIGGKQALETVQALLPVLCQAHGLTPQQVVAIASNGGGKQALETVQRLLPVLCQAHGL TPEQVVAIASNIGGKQALETVQALLPVLCQAHGLTPQQVVAIASNNGGKQALETVQRLLPVLCQAHGL TPEQVVAIASHDGGKQALETVQRLLPVLCQAHGLTPEQVVAIASNIGGKQALETVQALLPVLCQAHGL TPQQVVAIASNNGGKQALETVQRLLPVLCQAHGLTPEQVVAIASHDGGKQALETVQRLLPVLCQAHG LTPEQVVAIASNIGGKQALETVQALLPVLCQAHGLTPQQVVAIASNNGGKQALETVQRLLPVLCQAHG LTPQQVVAIASNGGGRPALESIVAQLSRPDPALAALTNDHLVALACLGGRPALDAVKKGLGDPISRSQ LVKSELEEKKSELRHKLKYVPHEYIELIEIARNSTQDRILEMKVMEFFMKVYGYRGKHLGGSRKPDGAI YTVGSPIDYGVIVDTKAYSGGYNLPIGQADEMQRYVEENQTRNKHINPNEWWKVYPSSVTEFKFLFV SGHFKGNYKAQLTRLNHITNCNGAVLSVEELLIGGEMIKAGTLTLEEVRRKFNNGEINFAAD |
| T002562 | HBV-polym-2.L1 | HD-HD-HD-NG-HD-NN-HD-HD-NG-HD-NN-HD-NI-NN-NI-NG# | (SEQ ID NO:73) CCCTCGCCTCG CAGAT# | (SEQ ID NO:15) MGDPKKKRKVIDYPYDVPDYAIDIADPIRSRTPSPARELLPGPQPDGVQPTADRGVSPPAGGPLDGLP ARRTMSRTRLPSPPAPSPAFSAGSFSDLLRQFDPSLFNTSLFDSLPPFGAHHTEAATGEWDEVQSGL RAADAPPPTMRVAVTAARPPRAKPAPRRRAAQPSDASPAAQVDLRTLGYSQQQQEKIKPKVRSTVA QHHEALVGHGFTHAHIVALSQHPAALGTVAVKYQDMIAALPEATHEAIVGVGKQWSGARALEALLTVA GELRGPPLQLDTGQLLKIAKRGGVTAVEAVHAWRNALTGAPLNLTPEQVVAIASHDGGKQALETVQR LLPVLCQAHGLTPEQVVAIASHDGGKQALETVQRLLPVLCQAHGLTPEQVVAIASHDGGKQALETVQ RLLPVLCQAHGLTPQQVVAIASNGGGKQALETVQRLLPVLCQAHGLTPEQVVAIASHDGGKQALETV QRLLPVLCQAHGLTPQQVVAIASNNGGKQALETVQRLLPVLCQAHGLTPEQVVAIASHDGGKQALET VQRLLPVLCQAHGLTPEQVVAIASHDGGKQALETVQRLLPVLCQAHGLTPQQVVAIASNGGGKQALE TVQRLLPVLCQAHGLTPEQVVAIASHDGGKQALETVQRLLPVLCQAHGLTPQQVVAIASNNGGKQAL ETVQRLLPVLCQAHGLTPEQVVAIASHDGGKQALETVQRLLPVLCQAHGLTPEQVVAIASNIGGKQAL ETVQALLPVLCQAHGLTPQQVVAIASNNGGKQALETVQRLLPVLCQAHGLTPEQVVAIASNIGGKQAL ETVQALLPVLCQAHGLTPQQVVAIASNGGGRPALESIVAQLSRPDPSGSGSGGDPISRSQLVKSELEE KKSELRHKLKYVPHEYIELIEIARNSTQDRILEMKVMEFFMKVYGYRGKHLGGSRKPDGAIYTVGSPID YGVIVDTKAYSGGYNLPIGQADEMQRYVEENQTRNKHINPNEWWKVYPSSVTEFKFLFVSGHFKGN YKAQLTRLNHITNCNGAVLSVEELLIGGEMIKAGTLTLEEVRRKFNNGEINFAAD |
| T002562 | HBV-polym-2.R1 | HD-NG-NG-HD-NG-NN-HD-NN-NI-HD-NN-HD-NN-NN-HD-NG# | (SEQ ID NO:74) CTTCTGCGACG CGGCT# | (SEQ ID NO:16) MGDPKKKRKVIDKETAAAKFERQHMDSIDIADPIRSRTPSPARELLPGPQPDGVQPTADRGVSPPAG GPLDGLPARRTMSRTRLPSPPAPSPAFSAGSFSDLLRQFDPSLFNTSLFDSLPPFGAHHTEAATGEW DEVQSGLRAADAPPPTMRVAVTAARPPRAKPAPRRRAAQPSDASPAAQVDLRTLGYSQQQQEKIKP KVRSTVAQHHEALVGHGFTHAHIVALSQHPAALGTVAVKYQDMIAALPEATHEAIVGVGKQWSGARA LEALLTVAGELRGPPLQLDTGQLLKIAKRGGVTAVEAVHAWRNALTGAPLNLTPEQVVAIASHDGGKQ ALETVQRLLPVLCQAHGLTPQQVVAIASNGGGKQALETVQRLLPVLCQAHGLTPQQVVAIASNGGGK QALETVQRLLPVLCQAHGLTPEQVVAIASHDGGKQALETVQRLLPVLCQAHGLTPQQVVAIASNGGG KQALETVQRLLPVLCQAHGLTPQQVVAIASNNGGKQALETVQRLLPVLCQAHGLTPEQVVAIASHDG GKQALETVQRLLPVLCQAHGLTPQQVVAIASNNGGKQALETVQRLLPVLCQAHGLTPEQVVAIASNIG GKQALETVQALLPVLCQAHGLTPEQVVAIASHDGGKQALETVQRLLPVLCQAHGLTPQQVVAIASNN GGKQALETVQRLLPVLCQAHGLTPEQVVAIASHDGGKQALETVQRLLPVLCQAHGLTPQQVVAIASN NGGKQALETVQRLLPVLCQAHGLTPQQVVAIASNNGGKQALETVQRLLPVLCQAHGLTPEQVVAIAS HDGGKQALETVQRLLPVLCQAHGLTPQQVVAIASNGGGRPALESIVAQLSRPDPSGSGSGGDPISRS QLVKSELEEKKSELRHKLKYVPHEYIELIEIARNSTQDRILEMKVMEFFMKVYGYRGKHLGGSRKPDG AIYTVGSPIDYGVIVDTKAYSGGYNLPIGQADEMQRYVEENQTRNKHINPNEWWKVYPSSVTEFKFLF VSGHFKGNYKAQLTRLNHITNCNGAVLSVEELLIGGEMIKAGTLTLEEVRRKFNNGEINFAAD |
| T002563 | HBV-HBX-1.L1 | NG-HD-NG-HD-NI-NG-HD-NG-NN-HD-HD-NN-NN-NG-HD-NG# | (SEQ ID NO:75) TCTCATCTGCC GGTCT# | (SEQ ID NO:17) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNGGGKQALETVQRLLPVLCQAHGLTPEQVVAI ASHDGGKQALETVQRLLPVLCQAHGLTPQQVVAIASNGGGKQALETVQRLLPVLCQAHGLTPEQVVA IASHDGGKQALETVQRLLPVLCQAHGLTPEQVVAIASNIGGKQALETVQALLPVLCQAHGLTPQQVVAI ASNGGGKQALETVQRLLPVLCQAHGLTPEQVVAIASHDGGKQALETVQRLLPVLCQAHGLTPQQVVA IASNGGGKQALETVQRLLPVLCQAHGLTPQQVVAIASNNGGKQALETVQRLLPVLCQAHGLTPEQVV AIASHDGGKQALETVQRLLPVLCQAHGLTPEQVVAIASHDGGKQALETVQRLLPVLCQAHGLTPQQV VAIASNNGGKQALETVQRLLPVLCQAHGLTPQQVVAIASNNGGKQALETVQRLLPVLCQAHGLTPQQ VVAIASNGGGKQALETVQRLLPVLCQAHGLTPEQVVAIASHDGGKQALETVQRLLPVLCQAHGLTPQ QVVAIASNGGGRPALESIVAQLSRPDPALAALTNDHLVALACLGGRPALDAVKKGLGDPISRSQLVKS ELEEKKSELRHKLKYVPHEYIELIEIARNSTQDRILEMKVMEFFMKVYGYRGKHLGGSRKPDGAIYTVG SPIDYGVIVDTKAYSGGYNLPIGQADEMQRYVEENQTRNKHINPNEWWKVYPSSVTEFKFLFVSGHF KGNYKAQLTRLNHITNCNGAVLSVEELLIGGEMIKAGTLTLEEVRRKFNNGEINFAAD |
| T002563 | HBV-HBX-1.R1 | NN-HD-NI-NI-HD-NN-NG-NN-HD-NI-NN-NI-NN-NN-NG-NG# | (SEQ ID NO:76) GCAACGTGCAG AGGTT# | (SEQ ID NO:18) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PEQVVAIASHDGGKQALETVQRLLPVLCQAHGLTPEQVVAIASNIGGKQALETVQALLPVLCQAHGLT PEQVVAIASNIGGKQALETVQALLPVLCQAHGLTPEQVVAIASHDGGKQALETVQRLLPVLCQAHGLT PQQVVAIASNNGGKQALETVQRLLPVLCQAHGLTPQQVVAIASNGGGKQALETVQRLLPVLCQAHGL TPQQVVAIASNNGGKQALETVQRLLPVLCQAHGLTPEQVVAIASHDGGKQALETVQRLLPVLCQAHG LTPEQVVAIASNIGGKQALETVQALLPVLCQAHGLTPQQVVAIASNNGGKQALETVQRLLPVLCQAHG LTPEQVVAIASNIGGKQALETVQALLPVLCQAHGLTPQQVVAIASNNGGKQALETVQRLLPVLCQAHG LTPQQVVAIASNNGGKQALETVQRLLPVLCQAHGLTPQQVVAIASNGGGKQALETVQRLLPVLCQAH GLTPQQVVAIASNGGGRPALESIVAQLSRPDPALAALTNDHLVALACLGGRPALDAVKKGLGDPISRS QLVKSELEEKKSELRHKLKYVPHEYIELIEIARNSTQDRILEMKVMEFFMKVYGYRGKHLGGSRKPDG AIYTVGSPIDYGVIVDTKAYSGGYNLPIGQADEMQRYVEENQTRNKHINPNEWWKVYPSSVTEFKFLF VSGHFKGNYKAQLTRLNHITNCNGAVLSVEELLIGGEMIKAGTLTLEEVRRKFNNGEINFAAD |
| T002564 | HBV-HBX-2.L1 | NG-NG-NI-HD-NN-HD-NN-NN-NG-HD-NG-HD-HD-HD-HD-NG# | (SEQ ID NO:77) TTACGCGGTCT CCCCT# | (SEQ ID NO:19) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNGGGKQALETVQRLLPVLCQAHGLTPQQVVAI ASNGGGKQALETVQRLLPVLCQAHGLTPEQVVAIASNIGGKQALETVQALLPVLCQAHGLTPEQVVAI ASHDGGKQALETVQRLLPVLCQAHGLTPQQVVAIASNNGGKQALETVQRLLPVLCQAHGLTPEQVVA IASHDGGKQALETVQRLLPVLCQAHGLTPQQVVAIASNNGGKQALETVQRLLPVLCQAHGLTPQQVV AIASNNGGKQALETVQRLLPVLCQAHGLTPQQVVAIASNGGGKQALETVQRLLPVLCQAHGLTPEQV VAIASHDGGKQALETVQRLLPVLCQAHGLTPQQVVAIASNGGGKQALETVQRLLPVLCQAHGLTPEQ VVAIASHDGGKQALETVQRLLPVLCQAHGLTPEQVVAIASHDGGKQALETVQRLLPVLCQAHGLTPE QVVAIASHDGGKQALETVQRLLPVLCQAHGLTPEQVVAIASHDGGKQALETVQRLLPVLCQAHGLTP QQVVAIASNGGGRPALESIVAQLSRPDPALAALTNDHLVALACLGGRPALDAVKKGLGDPISRSQLVK SELEEKKSELRHKLKYVPHEYIELIEIARNSTQDRILEMKVMEFFMKVYGYRGKHLGGSRKPDGAIYTV GSPIDYGVIVDTKAYSGGYNLPIGQADEMQRYVEENQTRNKHINPNEWWKVYPSSVTEFKFLFVSGH FKGNYKAQLTRLNHITNCNGAVLSVEELLIGGEMIKAGTLTLEEVRRKFNNGEINFAAD |
| T002564 | HBV-HBX-2.R1 | NN-HD-NI-HD-NI-HD-NN-NN-NI-HD-HD-NN-NN-HD-NI-NG# | (SEQ ID NO:78) GCACACGGACC GGCAT# | (SEQ ID NO:20) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PEQVVAIASHDGGKQALETVQRLLPVLCQAHGLTPEQVVAIASNIGGKQALETVQALLPVLCQAHGLT PEQVVAIASHDGGKQALETVQRLLPVLCQAHGLTPEQVVAIASNIGGKQALETVQALLPVLCQAHGLT PEQVVAIASHDGGKQALETVQRLLPVLCQAHGLTPQQVVAIASNNGGKQALETVQRLLPVLCQAHGL TPQQVVAIASNNGGKQALETVQRLLPVLCQAHGLTPEQVVAIASNIGGKQALETVQALLPVLCQAHGL TPEQVVAIASHDGGKQALETVQRLLPVLCQAHGLTPEQVVAIASHDGGKQALETVQRLLPVLCQAHG LTPQQVVAIASNNGGKQALETVQRLLPVLCQAHGLTPQQVVAIASNNGGKQALETVQRLLPVLCQAH GLTPEQVVAIASHDGGKQALETVQRLLPVLCQAHGLTPEQVVAIASNIGGKQALETVQALLPVLCQAH GLTPQQVVAIASNGGGRPALESIVAQLSRPDPALAALTNDHLVALACLGGRPALDAVKKGLGDPISRS QLVKSELEEKKSELRHKLKYVPHEYIELIEIARNSTQDRILEMKVMEFFMKVYGYRGKHLGGSRKPDG AIYTVGSPIDYGVIVDTKAYSGGYNLPIGQADEMQRYVEENQTRNKHINPNEWWKVYPSSVTEFKFLF VSGHFKGNYKAQLTRLNHITNCNGAVLSVEELLIGGEMIKAGTLTLEEVRRKFNNGEINFAAD |
| T002569 | mm_F7_exon1-3.L1 | HD-NG-HD-NG-NN-HD-NG-NG-NG-HD-NG-NN-HD-NG-HD-NG# | (SEQ ID NO:79) CTCTGCTTTCTG CTCT# | (SEQ ID NO:21) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPEQVVAIASHDGGKQALETVQRLLPVLCQAHGLTPQQVVAI ASNGGGKQALETVQRLLPVLCQAHGLTPEQVVAIASHDGGKQALETVQRLLPVLCQAHGLTPQQVVA IASNGGGKQALETVQRLLPVLCQAHGLTPQQVVAIASNNGGKQALETVQRLLPVLCQAHGLTPEQVV AIASHDGGKQALETVQRLLPVLCQAHGLTPQQVVAIASNGGGKQALETVQRLLPVLCQAHGLTPQQV VAIASNGGGKQALETVQRLLPVLCQAHGLTPQQVVAIASNGGGKQALETVQRLLPVLCQAHGLTPEQ VVAIASHDGGKQALETVQRLLPVLCQAHGLTPQQVVAIASNGGGKQALETVQRLLPVLCQAHGLTPQ QVVAIASNNGGKQALETVQRLLPVLCQAHGLTPEQVVAIASHDGGKQALETVQRLLPVLCQAHGLTP QQVVAIASNGGGKQALETVQRLLPVLCQAHGLTPEQVVAIASHDGGKQALETVQRLLPVLCQAHGLT PQQVVAIASNGGGRPALESIVAQLSRPDPALAALTNDHLVALACLGGRPALDAVKKGLGDPISRSQLV KSELEEKKSELRHKLKYVPHEYIELIEIARNSTQDRILEMKVMEFFMKVYGYRGKHLGGSRKPDGAIYT VGSPIDYGVIVDTKAYSGGYNLPIGQADEMQRYVEENQTRNKHINPNEWWKVYPSSVTEFKFLFVSG HFKGNYKAQLTRLNHITNCNGAVLSVEELLIGGEMIKAGTLTLEEVRRKFNNGEINFAAD |
| T002569 | mm_F7_exon1-3.R1 | NI-NG-NI-HD-HD-NG-NN-HD-NI-NN-NG-HD-HD-HD-NG-NG# | (SEQ ID NO:80) ATACCTGCAGT CCCTT# | (SEQ ID NO:22) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPEQVVAIASNIGGKQALETVQALLPVLCQAHGLTP QQVVAIASNGGGKQALETVQRLLPVLCQAHGLTPEQVVAIASNIGGKQALETVQALLPVLCQAHGLTP EQVVAIASHDGGKQALETVQRLLPVLCQAHGLTPEQVVAIASHDGGKQALETVQRLLPVLCQAHGLT PQQVVAIASNGGGKQALETVQRLLPVLCQAHGLTPQQVVAIASNNGGKQALETVQRLLPVLCQAHGL TPEQVVAIASHDGGKQALETVQRLLPVLCQAHGLTPEQVVAIASNIGGKQALETVQALLPVLCQAHGL TPQQVVAIASNNGGKQALETVQRLLPVLCQAHGLTPQQVVAIASNGGGKQALETVQRLLPVLCQAHG LTPEQVVAIASHDGGKQALETVQRLLPVLCQAHGLTPEQVVAIASHDGGKQALETVQRLLPVLCQAH GLTPEQVVAIASHDGGKQALETVQRLLPVLCQAHGLTPQQVVAIASNGGGKQALETVQRLLPVLCQA HGLTPQQVVAIASNGGGRPALESIVAQLSRPDPALAALTNDHLVALACLGGRPALDAVKKGLGDPISR SQLVKSELEEKKSELRHKLKYVPHEYIELIEIARNSTQDRILEMKVMEFFMKVYGYRGKHLGGSRKPD GAIYTVGSPIDYGVIVDTKAYSGGYNLPIGQADEMQRYVEENQTRNKHINPNEWWKVYPSSVTEFKFL FVSGHFKGNYKAQLTRLNHITNCNGAVLSVEELLIGGEMIKAGTLTLEEVRRKFNNGEINFAAD |
| T002575 | mm_F7_exon6-2.L1 | HD-NG-HD-NG-HD-NG-NN-NI-HD-NG-NG-HD-NG-NN-NG-NG# | (SEQ ID NO:81) CTCTCTGACTTC TGTT# | (SEQ ID NO:23) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPEQVVAIASHDGGKQALETVQRLLPVLCQAHGLTPQQVVAI ASNGGGKQALETVQRLLPVLCQAHGLTPEQVVAIASHDGGKQALETVQRLLPVLCQAHGLTPQQVVA IASNGGGKQALETVQRLLPVLCQAHGLTPEQVVAIASHDGGKQALETVQRLLPVLCQAHGLTPQQVV AIASNGGGKQALETVQRLLPVLCQAHGLTPQQVVAIASNNGGKQALETVQRLLPVLCQAHGLTPEQV VAIASNIGGKQALETVQALLPVLCQAHGLTPEQVVAIASHDGGKQALETVQRLLPVLCQAHGLTPQQV VAIASNGGGKQALETVQRLLPVLCQAHGLTPQQVVAIASNGGGKQALETVQRLLPVLCQAHGLTPEQ VVAIASHDGGKQALETVQRLLPVLCQAHGLTPQQVVAIASNGGGKQALETVQRLLPVLCQAHGLTPQ QVVAIASNNGGKQALETVQRLLPVLCQAHGLTPQQVVAIASNGGGKQALETVQRLLPVLCQAHGLTP QQVVAIASNGGGRPALESIVAQLSRPDPSGSGSGGDPISRSQLVKSELEEKKSELRHKLKYVPHEYIE LIEIARNSTQDRILEMKVMEFFMKVYGYRGKHLGGSRKPDGAIYTVGSPIDYGVIVDTKAYSGGYNLPI GQADEMQRYVEENQTRNKHINPNEWWKVYPSSVTEFKFLFVSGHFKGNYKAQLTRLNHITNCNGAV LSVEELLIGGEMIKAGTLTLEEVRRKFNNGEINFAAD |
| T002575 | mm_F7_exon6-2.R1 | NG-HD-NG-HD-NI-HD-NN-NN-NN-NG-NI-NG# | (SEQ ID NO:82) TCTCCCACACG GGTAT# | (SEQ ID NO:24) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNGGGKQALETVQRLLPVLCQAHGLT PEQVVAIASHDGGKQALETVQRLLPVLCQAHGLTPQQVVAIASNGGGKQALETVQRLLPVLCQAHGL TPEQVVAIASHDGGKQALETVQRLLPVLCQAHGLTPEQVVAIASHDGGKQALETVQRLLPVLCQAHG LTPEQVVAIASHDGGKQALETVQRLLPVLCQAHGLTPEQVVAIASNIGGKQALETVQALLPVLCQAHG LTPEQVVAIASHDGGKQALETVQRLLPVLCQAHGLTPEQVVAIASNIGGKQALETVQALLPVLCQAHG LTPEQVVAIASHDGGKQALETVQRLLPVLCQAHGLTPQQVVAIASNNGGKQALETVQRLLPVLCQAH GLTPQQVVAIASNNGGKQALETVQRLLPVLCQAHGLTPQQVVAIASNNGGKQALETVQRLLPVLCQA HGLTPQQVVAIASNGGGKQALETVQRLLPVLCQAHGLTPEQVVAIASNIGGKQALETVQALLPVLCQA HGLTPQQVVAIASNGGGRPALESIVAQLSRPDPSGSGSGGDPISRSQLVKSELEEKKSELRHKLKYVP HEYIELIEIARNSTQDRILEMKVMEFFMKVYGYRGKHLGGSRKPDGAIYTVGSPIDYGVIVDTKAYSGG YNLPIGQADEMQRYVEENQTRNKHINPNEWWKVYPSSVTEFKFLFVSGHFKGNYKAQLTRLNHITNC NGAVLSVEELLIGGEMIKAGTLTLEEVRRKFNNGEINFAAD |
| T002657 | hAPOC3_Ex3A-L1 | HD-NG-NN-NG-NG-NN-HD-NG-NG-HD-HD-HD-HD-NG-NN-NG# | (SEQ ID NO:83) CTGTTGCTTCCC CTGT# | (SEQ ID NO:25) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPEQVVAIASHDGGKQALETVQRLLPVLCQAHGLTPQQVVAI ASNGGGKQALETVQRLLPVLCQAHGLTPQQVVAIASNNGGKQALETVQRLLPVLCQAHGLTPQQVV AIASNGGGKQALETVQRLLPVLCQAHGLTPQQVVAIASNGGGKQALETVQRLLPVLCQAHGLTPQQV VAIASNNGGKQALETVQRLLPVLCQAHGLTPEQVVAIASHDGGKQALETVQRLLPVLCQAHGLTPQQ VVAIASNGGGKQALETVQRLLPVLCQAHGLTPQQVVAIASNGGGKQALETVQRLLPVLCQAHGLTPE QVVAIASHDGGKQALETVQRLLPVLCQAHGLTPEQVVAIASHDGGKQALETVQRLLPVLCQAHGLTP EQVVAIASHDGGKQALETVQRLLPVLCQAHGLTPEQVVAIASHDGGKQALETVQRLLPVLCQAHGLT PQQVVAIASNGGGKQALETVQRLLPVLCQAHGLTPQQVVAIASNNGGKQALETVQRLLPVLCQAHGL TPQQVVAIASNGGGRPALESIVAQLSRPDPALAALTNDHLVALACLGGRPALDAVKKGLGDPISRSQL VKSELEEKKSELRHKLKYVPHEYIELIEIARNSTQDRILEMKVMEFFMKVYGYRGKHLGGSRKPDGAIY TVGSPIDYGVIVDTKAYSGGYNLPIGQADEMQRYVEENQTRNKHINPNEWWKVYPSSVTEFKFLFVS GHFKGNYKAQLTRLNHITNCNGAVLSVEELLIGGEMIKAGTLTLEEVRRKFNNGEINFAAD |
| T002657 | hAPOC3_Ex3A-R1 | NN-NI-NI-NN-HD-HD-NI-NG-HD-NN-NN-NG-HD-NI-HD-NG# | (SEQ ID NO:84) GAAGCCATCGG TCACT# | (SEQ ID NO:26) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PEQVVAIASNIGGKQALETVQALLPVLCQAHGLTPEQVVAIASNIGGKQALETVQALLPVLCQAHGLTP QQVVAIASNNGGKQALETVQRLLPVLCQAHGLTPEQVVAIASHDGGKQALETVQRLLPVLCQAHGLT PEQVVAIASHDGGKQALETVQRLLPVLCQAHGLTPEQVVAIASNIGGKQALETVQALLPVLCQAHGLT PQQVVAIASNGGGKQALETVQRLLPVLCQAHGLTPEQVVAIASHDGGKQALETVQRLLPVLCQAHGL TPQQVVAIASNNGGKQALETVQRLLPVLCQAHGLTPQQVVAIASNNGGKQALETVQRLLPVLCQAHG LTPQQVVAIASNGGGKQALETVQRLLPVLCQAHGLTPEQVVAIASHDGGKQALETVQRLLPVLCQAH GLTPEQVVAIASNIGGKQALETVQALLPVLCQAHGLTPEQVVAIASHDGGKQALETVQRLLPVLCQAH GLTPQQVVAIASNGGGRPALESIVAQLSRPDPALAALTNDHLVALACLGGRPALDAVKKGLGDPISRS QLVKSELEEKKSELRHKLKYVPHEYIELIEIARNSTQDRILEMKVMEFFMKVYGYRGKHLGGSRKPDG AIYTVGSPIDYGVIVDTKAYSGGYNLPIGQADEMQRYVEENQTRNKHINPNEWWKVYPSSVTEFKFLF VSGHFKGNYKAQLTRLNHITNCNGAVLSVEELLIGGEMIKAGTLTLEEVRRKFNNGEINFAAD |
| T002658 | hAPOC3_Ex3B-L1 | NN-NI-NI-NI-NN-NI-HD-NG-NI-HD-NG-NN-NN-NI-NN-NG# | (SEQ ID NO:85) GAAAGACTACT GGAGT# | (SEQ ID NO:27) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNNGGKQALETVQRLLPVLCQAHGLTPEQVVAI ASNIGGKQALETVQALLPVLCQAHGLTPEQVVAIASNIGGKQALETVQALLPVLCQAHGLTPEQVVAIA SNIGGKQALETVQALLPVLCQAHGLTPQQVVAIASNNGGKQALETVQRLLPVLCQAHGLTPEQVVAIA SNIGGKQALETVQALLPVLCQAHGLTPEQVVAIASHDGGKQALETVQRLLPVLCQAHGLTPQQVVAIA SNGGGKQALETVQRLLPVLCQAHGLTPEQVVAIASNIGGKQALETVQALLPVLCQAHGLTPEQVVAIA SHDGGKQALETVQRLLPVLCQAHGLTPQQVVAIASNGGGKQALETVQRLLPVLCQAHGLTPQQVVAI ASNNGGKQALETVQRLLPVLCQAHGLTPQQVVAIASNNGGKQALETVQRLLPVLCQAHGLTPEQVVA IASNIGGKQALETVQALLPVLCQAHGLTPQQVVAIASNNGGKQALETVQRLLPVLCQAHGLTPQQVVA IASNGGGRPALESIVAQLSRPDPALAALTNDHLVALACLGGRPALDAVKKGLGDPISRSQLVKSELEEK KSELRHKLKYVPHEYIELIEIARNSTQDRILEMKVMEFFMKVYGYRGKHLGGSRKPDGAIYTVGSPIDY GVIVDTKAYSGGYNLPIGQADEMQRYVEENQTRNKHINPNEWWKVYPSSVTEFKFLFVSGHFKGNY KAQLTRLNHITNCNGAVLSVEELLIGGEMIKAGTLTLEEVRRKFNNGEINFAAD |
| T002658 | hAPOC3_Ex3B-R1 | HD-HD-HD-NI-NN-NI-NI-HD-NG-HD-NI-NN-NI-NN-NI-NG# | (SEQ ID NO:86) CCCAGAACTCA GAGAT# | (SEQ ID NO:28) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPEQVVAIASHDGGKQALETVQRLLPVLCQAHGLT PEQVVAIASHDGGKQALETVQRLLPVLCQAHGLTPEQVVAIASHDGGKQALETVQRLLPVLCQAHGL TPEQVVAIASNIGGKQALETVQALLPVLCQAHGLTPQQVVAIASNNGGKQALETVQRLLPVLCQAHGL TPEQVVAIASNIGGKQALETVQALLPVLCQAHGLTPEQVVAIASNIGGKQALETVQALLPVLCQAHGLT PEQVVAIASHDGGKQALETVQRLLPVLCQAHGLTPQQVVAIASNGGGKQALETVQRLLPVLCQAHGL TPEQVVAIASHDGGKQALETVQRLLPVLCQAHGLTPEQVVAIASNIGGKQALETVQALLPVLCQAHGL TPQQVVAIASNNGGKQALETVQRLLPVLCQAHGLTPEQVVAIASNIGGKQALETVQALLPVLCQAHGL TPQQVVAIASNNGGKQALETVQRLLPVLCQAHGLTPEQVVAIASNIGGKQALETVQALLPVLCQAHGL TPQQVVAIASNGGGRPALESIVAQLSRPDPALAALTNDHLVALACLGGRPALDAVKKGLGDPISRSQL VKSELEEKKSELRHKLKYVPHEYIELIEIARNSTQDRILEMKVMEFFMKVYGYRGKHLGGSRKPDGAIY TVGSPIDYGVIVDTKAYSGGYNLPIGQADEMQRYVEENQTRNKHINPNEWWKVYPSSVTEFKFLFVS GHFKGNYKAQLTRLNHITNCNGAVLSVEELLIGGEMIKAGTLTLEEVRRKFNNGEINFAAD |
| T002671 | hAPOC3_Ex3_AN-R1 | NN-NI-NI-NN-HD-HD-NI-NG-N-NN-NN-NG-HD-NI-HD-NG# | (SEQ ID NO:87) GAAGCCAT5GG TCACT# | (SEQ ID NO:29) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PEQVVAIASNIGGKQALETVQALLPVLCQAHGLTPEQVVAIASNIGGKQALETVQALLPVLCQAHGLTP QQVVAIASNNGGKQALETVQRLLPVLCQAHGLTPEQVVAIASHDGGKQALETVQRLLPVLCQAHGLT PEQVVAIASHDGGKQALETVQRLLPVLCQAHGLTPEQVVAIASNIGGKQALETVQALLPVLCQAHGLT PQQVVAIASNGGGKQALETVQRLLPVLCQAHGLTPQQVVAIASNGGKQALETVQRLLPVLCQAHGLT PQQVVAIASNNGGKQALETVQRLLPVLCQAHGLTPQQVVAIASNNGGKQALETVQRLLPVLCQAHGL TPQQVVAIASNGGGKQALETVQRLLPVLCQAHGLTPEQVVAIASHDGGKQALETVQRLLPVLCQAHG LTPEQVVAIASNIGGKQALETVQALLPVLCQAHGLTPEQVVAIASHDGGKQALETVQRLLPVLCQAHG LTPQQVVAIASNGGGRPALESIVAQLSRPDPALAALTNDHLVALACLGGRPALDAVKKGLGDPISRSQ LVKSELEEKKSELRHKLKYVPHEYIELIEIARNSTQDRILEMKVMEFFMKVYGYRGKHLGGSRKPDGAI YTVGSPIDYGVIVDTKAYSGGYNLPIGQADEMQRYVEENQTRNKHINPNEWWKVYPSSVTEFKFLFV SGHFKGNYKAQLTRLNHITNCNGAVLSVEELLIGGEMIKAGTLTLEEVRRKFNNGEINFAAD |
| T002671 | hAPOC3_Ex3A-L1 | HD-NG-NN-NG-NG-NN-HD-NG-NG-HD-HD-HD-HD-NG-NN-NG# | (SEQ ID NO:88) CTGTTGCTTCCC CTGT# | (SEQ ID NO:30) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPEQVVAIASHDGGKQALETVQRLLPVLCQAHGLTPQQVVAI ASNGGGKQALETVQRLLPVLCQAHGLTPQQVVAIASNNGGKQALETVQRLLPVLCQAHGLTPQQVV AIASNGGGKQALETVQRLLPVLCQAHGLTPQQVVAIASNGGGKQALETVQRLLPVLCQAHGLTPQQV VAIASNNGGKQALETVQRLLPVLCQAHGLTPEQVVAIASHDGGKQALETVQRLLPVLCQAHGLTPQQ VVAIASNGGGKQALETVQRLLPVLCQAHGLTPQQVVAIASNGGGKQALETVQRLLPVLCQAHGLTPE QVVAIASHDGGKQALETVQRLLPVLCQAHGLTPEQVVAIASHDGGKQALETVQRLLPVLCQAHGLTP EQVVAIASHDGGKQALETVQRLLPVLCQAHGLTPEQVVAIASHDGGKQALETVQRLLPVLCQAHGLT PQQVVAIASNGGGKQALETVQRLLPVLCQAHGLTPQQVVAIASNNGGKQALETVQRLLPVLCQAHGL TPQQVVAIASNGGGRPALESIVAQLSRPDPALAALTNDHLVALACLGGRPALDAVKKGLGDPISRSQL VKSELEEKKSELRHKLKYVPHEYIELIEIARNSTQDRILEMKVMEFFMKVYGYRGKHLGGSRKPDGAIY TVGSPIDYGVIVDTKAYSGGYNLPIGQADEMQRYVEENQTRNKHINPNEWWKVYPSSVTEFKFLFVS GHFKGNYKAQLTRLNHITNCNGAVLSVEELLIGGEMIKAGTLTLEEVRRKFNNGEINFAAD |
| T002659 | hTTR_Ex1A-L1 | HD-NG-NG-NN-NN-HD-NI-NN-NN-NI-NG-NN-NN-HD-NG-NG# | (SEQ ID NO:89) CTTGGCAGGAT GGCTT# | (SEQ ID NO:31) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPEQVVAIASHDGGKQALETVQRLLPVLCQAHGLTPQQVVAI ASNGGGKQALETVQRLLPVLCQAHGLTPQQVVAIASNGGGKQALETVQRLLPVLCQAHGLTPQQVV AIASNNGGKQALETVQRLLPVLCQAHGLTPQQVVAIASNNGGKQALETVQRLLPVLCQAHGLTPEQV VAIASHDGGKQALETVQRLLPVLCQAHGLTPEQVVAIASNIGGKQALETVQALLPVLCQAHGLTPQQV VAIASNNGGKQALETVQRLLPVLCQAHGLTPQQVVAIASNNGGKQALETVQRLLPVLCQAHGLTPEQ VVAIASNIGGKQALETVQALLPVLCQAHGLTPQQVVAIASNGGGKQALETVQRLLPVLCQAHGLTPQQ VVAIASNNGGKQALETVQRLLPVLCQAHGLTPQQVVAIASNNGGKQALETVQRLLPVLCQAHGLTPE QVVAIASHDGGKQALETVQRLLPVLCQAHGLTPQQVVAIASNGGGKQALETVQRLLPVLCQAHGLTP QQVVAIASNGGGRPALESIVAQLSRPDPALAALTNDHLVALACLGGRPALDAVKKGLGDPISRSQLVK SELEEKKSELRHKLKYVPHEYIELIEIARNSTQDRILEMKVMEFFMKVYGYRGKHLGGSRKPDGAIYTV GSPIDYGVIVDTKAYSGGYNLPIGQADEMQRYVEENQTRNKHINPNEWWKVYPSSVTEFKFLFVSGH FKGNYKAQLTRLNHITNCNGAVLSVEELLIGGEMIKAGTLTLEEVRRKFNNGEINFAAD |
| T002659 | hTTR_Ex1A-R1 | HD-HD-NI-NN-HD-NI-NI-NN-NN-HD-NI-NN-NI-NN-NN-NG# | (SEQ ID NO:90) CCAGCAAGGCA GAGGT# | (SEQ ID NO:32) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPEQVVAIASHDGGKQALETVQRLLPVLCQAHGLT PEQVVAIASHDGGKQALETVQRLLPVLCQAHGLTPEQVVAIASNIGGKQALETVQALLPVLCQAHGLT PQQVVAIASNNGGKQALETVQRLLPVLCQAHGLTPEQVVAIASHDGGKQALETVQRLLPVLCQAHGL TPEQVVAIASNIGGKQALETVQALLPVLCQAHGLTPEQVVAIASNIGGKQALETVQALLPVLCQAHGLT PQQVVAIASNNGGKQALETVQRLLPVLCQAHGLTPQQVVAIASNNGGKQALETVQRLLPVLCQAHGL TPEQVVAIASHDGGKQALETVQRLLPVLCQAHGLTPEQVVAIASNIGGKQALETVQALLPVLCQAHGL TPQQVVAIASNNGGKQALETVQRLLPVLCQAHGLTPEQVVAIASNIGGKQALETVQALLPVLCQAHGL TPQQVVAIASNNGGKQALETVQRLLPVLCQAHGLTPQQVVAIASNNGGKQALETVQRLLPVLCQAHG LTPQQVVAIASNGGGRPALESIVAQLSRPDPALAALTNDHLVALACLGGRPALDAVKKGLGDPISRSQ LVKSELEEKKSELRHKLKYVPHEYIELIEIARNSTQDRILEMKVMEFFMKVYGYRGKHLGGSRKPDGAI YTVGSPIDYGVIVDTKAYSGGYNLPIGQADEMQRYVEENQTRNKHINPNEWWKVYPSSVTEFKFLFV SGHFKGNYKAQLTRLNHITNCNGAVLSVEELLIGGEMIKAGTLTLEEVRRKFNNGEINFAAD |
| T002660 | hTTR_Ex1B-L1 | NN-NG-HD-NG-NN-NI-NN-NN-HD-NG-NN-NN-HD-HD-HD-NG# | (SEQ ID NO:91) GTCTGAGGCTG GCCCT# | (SEQ ID NO:33) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNNGGKQALETVQRLLPVLCQAHGLTPQQVVAI ASNGGGKQALETVQRLLPVLCQAHGLTPEQVVAIASHDGGKQALETVQRLLPVLCQAHGLTPQQVVA IASNGGGKQALETVQRLLPVLCQAHGLTPQQVVAIASNNGGKQALETVQRLLPVLCQAHGLTPEQVV AIASNIGGKQALETVQALLPVLCQAHGLTPQQVVAIASNNGGKQALETVQRLLPVLCQAHGLTPQQVV AIASNNGGKQALETVQRLLPVLCQAHGLTPEQVVAIASHDGGKQALETVQRLLPVLCQAHGLTPQQV VAIASNGGGKQALETVQRLLPVLCQAHGLTPQQVVAIASNNGGKQALETVQRLLPVLCQAHGLTPQQ VVAIASNNGGKQALETVQRLLPVLCQAHGLTPEQVVAIASHDGGKQALETVQRLLPVLCQAHGLTPE QVVAIASHDGGKQALETVQRLLPVLCQAHGLTPEQVVAIASHDGGKQALETVQRLLPVLCQAHGLTP QQVVAIASNGGGRPALESIVAQLSRPDPALAALTNDHLVALACLGGRPALDAVKKGLGDPISRSQLVK SELEEKKSELRHKLKYVPHEYIELIEIARNSTQDRILEMKVMEFFMKVYGYRGKHLGGSRKPDGAIYTV GSPIDYGVIVDTKAYSGGYNLPIGQADEMQRYVEENQTRNKHINPNEWWKVYPSSVTEFKFLFVSGH FKGNYKAQLTRLNHITNCNGAVLSVEELLIGGEMIKAGTLTLEEVRRKFNNGEINFAAD |
| T002660 | hTTR_Ex1B-R1 | NI-NN-NN-NI-NI-NG-NN-NN-NN-NI-NG-NN-NG-HD-NI-NG# | (SEQ ID NO:92) AGGAATGGGAT GTCAT# | (SEQ ID NO:34) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPEQVVAIASNIGGKQALETVQALLPVLCQAHGLTP QQVVAIASNNGGKQALETVQRLLPVLCQAHGLTPQQVVAIASNNGGKQALETVQRLLPVLCQAHGLT PEQVVAIASNIGGKQALETVQALLPVLCQAHGLTPEQVVAIASNIGGKQALETVQALLPVLCQAHGLTP QQVVAIASNGGGKQALETVQRLLPVLCQAHGLTPQQVVAIASNNGGKQALETVQRLLPVLCQAHGLT PQQVVAIASNNGGKQALETVQRLLPVLCQAHGLTPQQVVAIASNNGGKQALETVQRLLPVLCQAHGL TPEQVVAIASNIGGKQALETVQALLPVLCQAHGLTPQQVVAIASNGGGKQALETVQRLLPVLCQAHGL TPQQVVAIASNNGGKQALETVQRLLPVLCQAHGLTPQQVVAIASNGGGKQALETVQRLLPVLCQAHG LTPEQVVAIASHDGGKQALETVQRLLPVLCQAHGLTPEQVVAIASNIGGKQALETVQALLPVLCQAHG LTPQQVVAIASNGGGRPALESIVAQLSRPDPALAALTNDHLVALACLGGRPALDAVKKGLGDPISRSQ LVKSELEEKKSELRHKLKYVPHEYIELIEIARNSTQDRILEMKVMEFFMKVYGYRGKHLGGSRKPDGAI YTVGSPIDYGVIVDTKAYSGGYNLPIGQADEMQRYVEENQTRNKHINPNEWWKVYPSSVTEFKFLFV SGHFKGNYKAQLTRLNHITNCNGAVLSVEELLIGGEMIKAGTLTLEEVRRKFNNGEINFAAD |
| T002661 | hSMN2_3ssEx8A-L1 | HD-NG-NN-NN-NG-NG-HD-NG-NI-NI-NG-NG-NG-HD-NG-NG# | (SEQ ID NO:93) CTGGTTCTAATT TCTT# | (SEQ ID NO:35) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPEQVVAIASHDGGKQALETVQRLLPVLCQAHGLTPQQVVAI ASNGGGKQALETVQRLLPVLCQAHGLTPQQVVAIASNNGGKQALETVQRLLPVLCQAHGLTPQQVV AIASNNGGKQALETVQRLLPVLCQAHGLTPQQVVAIASNGGGKQALETVQRLLPVLCQAHGLTPQQV VAIASNGGGKQALETVQRLLPVLCQAHGLTPEQVVAIASHDGGKQALETVQRLLPVLCQAHGLTPQQ VVAIASNGGGKQALETVQRLLPVLCQAHGLTPEQVVAIASNIGGKQALETVQALLPVLCQAHGLTPEQ VVAIASNIGGKQALETVQALLPVLCQAHGLTPQQVVAIASNGGGKQALETVQRLLPVLCQAHGLTPQQ VVAIASNGGGKQALETVQRLLPVLCQAHGLTPQQVVAIASNGGGKQALETVQRLLPVLCQAHGLTPE QVVAIASHDGGKQALETVQRLLPVLCQAHGLTPQQVVAIASNGGGKQALETVQRLLPVLCQAHGLTP QQVVAIASNGGGRPALESIVAQLSRPDPSGSGSGGDPISRSQLVKSELEEKKSELRHKLKYVPHEYIE LIEIARNSTQDRILEMKVMEFFMKVYGYRGKHLGGSRKPDGAIYTVGSPIDYGVIVDTKAYSGGYNLPI GQADEMQRYVEENQTRNKHINPNEWWKVYPSSVTEFKFLFVSGHFKGNYKAQLTRLNHITNCNGAV LSVEELLIGGEMIKAGTLTLEEVRRKFNNGEINFAAD |
| T002661 | hSMN2_3ssEx8A-R1 | NN-HD-NG-NN-HD-NG-HD-NG-NI-NG-NN-HD-HD-NI-NN-NG# | (SEQ ID NO:94) GCTGCTCTATG CCAGT# | (SEQ ID NO:36) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PEQVVAIASHDGGKQALETVQRLLPVLCQAHGLTPQQVVAIASNGGGKQALETVQRLLPVLCQAHGL TPQQVVAIASNNGGKQALETVQRLLPVLCQAHGLTPEQVVAIASHDGGKQALETVQRLLPVLCQAHG LTPQQVVAIASNGGGKQALETVQRLLPVLCQAHGLTPEQVVAIASHDGGKQALETVQRLLPVLCQAH GLTPQQVVAIASNGGGKQALETVQRLLPVLCQAHGLTPEQVVAIASNIGGKQALETVQALLPVLCQAH GLTPQQVVAIASNGGGKQALETVQRLLPVLCQAHGLTPQQVVAIASNNGGKQALETVQRLLPVLCQA HGLTPEQVVAIASHDGGKQALETVQRLLPVLCQAHGLTPEQVVAIASHDGGKQALETVQRLLPVLCQ AHGLTPEQVVAIASNIGGKQALETVQALLPVLCQAHGLTPQQVVAIASNNGGKQALETVQRLLPVLCQ AHGLTPQQVVAIASNGGGRPALESIVAQLSRPDPSGSGSGGDPISRSQLVKSELEEKKSELRHKLKYV PHEYIELIEIARNSTQDRILEMKVMEFFMKVYGYRGKHLGGSRKPDGAIYTVGSPIDYGVIVDTKAYSG GYNLPIGQADEMQRYVEENQTRNKHINPNEWWKVYPSSVTEFKFLFVSGHFKGNYKAQLTRLNHITN CNGAVLSVEELLIGGEMIKAGTLTLEEVRRKFNNGEINFAAD |
| T002662 | hSMN2_3ssEx8B-L1 | NN-NN-NG-NG-HD-NG-NI-NI-NG-NG-NG-HD-NG-HD-NI-NG# | (SEQ ID NO:95) GGTTCTAATTTC TCAT# | (SEQ ID NO:37) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNNGGKQALETVQRLLPVLCQAHGLTPQQVVAI ASNNGGKQALETVQRLLPVLCQAHGLTPQQVVAIASNGGGKQALETVQRLLPVLCQAHGLTPQQVV AIASNGGGKQALETVQRLLPVLCQAHGLTPEQVVAIASHDGGKQALETVQRLLPVLCQAHGLTPQQV VAIASNGGGKQALETVQRLLPVLCQAHGLTPEQVVAIASNIGGKQALETVQALLPVLCQAHGLTPEQV VAIASNIGGKQALETVQALLPVLCQAHGLTPQQVVAIASNGGGKQALETVQRLLPVLCQAHGLTPQQV VAIASNGGGKQALETVQRLLPVLCQAHGLTPQQVVAIASNGGGKQALETVQRLLPVLCQAHGLTPEQ VVAIASHDGGKQALETVQRLLPVLCQAHGLTPQQVVAIASNGGGKQALETVQRLLPVLCQAHGLTPE QVVAIASHDGGKQALETVQRLLPVLCQAHGLTPEQVVAIASNIGGKQALETVQALLPVLCQAHGLTPQ QVVAIASNGGGRPALESIVAQLSRPDPALAALTNDHLVALACLGGRPALDAVKKGLGDPISRSQLVKS ELEEKKSELRHKLKYVPHEYIELIEIARNSTQDRILEMKVMEFFMKVYGYRGKHLGGSRKPDGAIYTVG SPIDYGVIVDTKAYSGGYNLPIGQADEMQRYVEENQTRNKHINPNEWWKVYPSSVTEFKFLFVSGHF KGNYKAQLTRLNHITNCNGAVLSVEELLIGGEMIKAGTLTLEEVRRKFNNGEINFAAD |
| T002662 | hSMN2_3ssEx8B-R1 | NG-NI-NN-NG-NN-HD-NG-NN-HD-NG-HD-NG-NI-NG-NN-NG# | (SEQ ID NO:96) TAGTGCTGCTCT ATGT# | (SEQ ID NO:38) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNGGGKQALETVQRLLPVLCQAHGLT PEQVVAIASNIGGKQALETVQALLPVLCQAHGLTPQQVVAIASNNGGKQALETVQRLLPVLCQAHGLT PQQVVAIASNGGGKQALETVQRLLPVLCQAHGLTPQQVVAIASNNGGKQALETVQRLLPVLCQAHGL TPEQVVAIASHDGGKQALETVQRLLPVLCQAHGLTPQQVVAIASNGGGKQALETVQRLLPVLCQAHG LTPQQVVAIASNNGGKQALETVQRLLPVLCQAHGLTPEQVVAIASHDGGKQALETVQRLLPVLCQAH GLTPQQVVAIASNGGGKQALETVQRLLPVLCQAHGLTPEQVVAIASHDGGKQALETVQRLLPVLCQA HGLTPQQVVAIASNGGGKQALETVQRLLPVLCQAHGLTPEQVVAIASNIGGKQALETVQALLPVLCQA HGLTPQQVVAIASNGGGKQALETVQRLLPVLCQAHGLTPQQVVAIASNNGGKQALETVQRLLPVLCQ AHGLTPQQVVAIASNGGGRPALESIVAQLSRPDPALAALTNDHLVALACLGGRPALDAVKKGLGDPIS RSQLVKSELEEKKSELRHKLKYVPHEYIELIEIARNSTQDRILEMKVMEFFMKVYGYRGKHLGGSRKP DGAIYTVGSPIDYGVIVDTKAYSGGYNLPIGQADEMQRYVEENQTRNKHINPNEWWKVYPSSVTEFK FLFVSGHFKGNYKAQLTRLNHITNCNGAVLSVEELLIGGEMIKAGTLTLEEVRRKFNNGEINFAAD |
| T002663 | hSMN2_ISS100A-L1 | NG-HD-NI-NN-NI-NG-NN-NG-NG-NI-NN-NI-NI-NI-NN-NG# | (SEQ ID NO:97) TCAGATGTTAGA AAGT# | (SEQ ID NO:39) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNGGGKQALETVQRLLPVLCQAHGLTPEQVVAI ASHDGGKQALETVQRLLPVLCQAHGLTPEQVVAIASNIGGKQALETVQALLPVLCQAHGLTPQQVVAI ASNNGGKQALETVQRLLPVLCQAHGLTPEQVVAIASNIGGKQALETVQALLPVLCQAHGLTPQQVVAI ASNGGGKQALETVQRLLPVLCQAHGLTPQQVVAIASNNGGKQALETVQRLLPVLCQAHGLTPQQVV AIASNGGGKQALETVQRLLPVLCQAHGLTPQQVVAIASNGGGKQALETVQRLLPVLCQAHGLTPEQV VAIASNIGGKQALETVQALLPVLCQAHGLTPQQVVAIASNNGGKQALETVQRLLPVLCQAHGLTPEQV VAIASNIGGKQALETVQALLPVLCQAHGLTPEQVVAIASNIGGKQALETVQALLPVLCQAHGLTPEQVV AIASNIGGKQALETVQALLPVLCQAHGLTPQQVVAIASNNGGKQALETVQRLLPVLCQAHGLTPQQVV AIASNGGGRPALESIVAQLSRPDPALAALTNDHLVALACLGGRPALDAVKKGLGDPISRSQLVKSELEE KKSELRHKLKYVPHEYIELIEIARNSTQDRILEMKVMEFFMKVYGYRGKHLGGSRKPDGAIYTVGSPID YGVIVDTKAYSGGYNLPIGQADEMQRYVEENQTRNKHINPNEWWKVYPSSVTEFKFLFVSGHFKGN YKAQLTRLNHITNCNGAVLSVEELLIGGEMIKAGTLTLEEVRRKFNNGEINFAAD |
| T002663 | hSMN2_ISS100A-R1 | NG-NG-NI-NI-NG-NI-NG-NG-NN-NI-NG-NG-NN-NG-NG-NG# | (SEQ ID NO:98) TTAATATTGATT GTTT# | (SEQ ID NO:40) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNGGGKQALETVQRLLPVLCQAHGLT PQQVVAIASNGGGKQALETVQRLLPVLCQAHGLTPEQVVAIASNIGGKQALETVQALLPVLCQAHGLT PEQVVAIASNIGGKQALETVQALLPVLCQAHGLTPQQVVAIASNGGGKQALETVQRLLPVLCQAHGLT PEQVVAIASNIGGKQALETVQALLPVLCQAHGLTPQQVVAIASNGGGKQALETVQRLLPVLCQAHGLT PQQVVAIASNGGGKQALETVQRLLPVLCQAHGLTPQQVVAIASNNGGKQALETVQRLLPVLCQAHGL TPEQVVAIASNIGGKQALETVQALLPVLCQAHGLTPQQVVAIASNGGGKQALETVQRLLPVLCQAHGL TPQQVVAIASNGGGKQALETVQRLLPVLCQAHGLTPQQVVAIASNNGGKQALETVQRLLPVLCQAHG LTPQQVVAIASNGGGKQALETVQRLLPVLCQAHGLTPQQVVAIASNGGGKQALETVQRLLPVLCQAH GLTPQQVVAIASNGGGRPALESIVAQLSRPDPALAALTNDHLVALACLGGRPALDAVKKGLGDPISRS QLVKSELEEKKSELRHKLKYVPHEYIELIEIARNSTQDRILEMKVMEFFMKVYGYRGKHLGGSRKPDG AIYTVGSPIDYGVIVDTKAYSGGYNLPIGQADEMQRYVEENQTRNKHINPNEWWKVYPSSVTEFKFLF VSGHFKGNYKAQLTRLNHITNCNGAVLSVEELLIGGEMIKAGTLTLEEVRRKFNNGEINFAAD |
| T002664 | hSMN2_ISS-N1A-L1 | NG-NI-NI-NN-NN-NI-NN-NG-NI-NI-NN-NG-HD-NG-NN-NG# | (SEQ ID NO:99) TAAGGAGTAAG TCTGT# | (SEQ ID NO:41) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNGGGKQALETVQRLLPVLCQAHGLTPEQVVAI ASNIGGKQALETVQALLPVLCQAHGLTPEQVVAIASNIGGKQALETVQALLPVLCQAHGLTPQQVVAIA SNNGGKQALETVQRLLPVLCQAHGLTPQQVVAIASNNGGKQALETVQRLLPVLCQAHGLTPEQVVAI ASNIGGKQALETVQALLPVLCQAHGLTPQQVVAIASNNGGKQALETVQRLLPVLCQAHGLTPQQVVAI ASNGGGKQALETVQRLLPVLCQAHGLTPEQVVAIASNIGGKQALETVQALLPVLCQAHGLTPEQVVAI ASNIGGKQALETVQALLPVLCQAHGLTPQQVVAIASNNGGKQALETVQRLLPVLCQAHGLTPQQVVAI ASNGGGKQALETVQRLLPVLCQAHGLTPEQVVAIASHDGGKQALETVQRLLPVLCQAHGLTPQQVVA IASNGGGKQALETVQRLLPVLCQAHGLTPQQVVAIASNNGGKQALETVQRLLPVLCQAHGLTPQQVV AIASNGGGRPALESIVAQLSRPDPALAALTNDHLVALACLGGRPALDAVKKGLGDPISRSQLVKSELEE KKSELRHKLKYVPHEYIELIEIARNSTQDRILEMKVMEFFMKVYGYRGKHLGGSRKPDGAIYTVGSPID YGVIVDTKAYSGGYNLPIGQADEMQRYVEENQTRNKHINPNEWWKVYPSSVTEFKFLFVSGHFKGN YKAQLTRLNHITNCNGAVLSVEELLIGGEMIKAGTLTLEEVRRKFNNGEINFAAD |
| T002664 | hSMN2_ISS-N1A-R1 | NG-NI-HD-NI-NI-NI-NI-NN-NG-NI-NI-NN-NI-NG-NG-NG# | (SEQ ID NO:100) TACAAAAGTAAG ATTT# | (SEQ ID NO:42) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNGGGKQALETVQRLLPVLCQAHGLT PEQVVAIASNIGGKQALETVQALLPVLCQAHGLTPEQVVAIASHDGGKQALETVQRLLPVLCQAHGLT PEQVVAIASNIGGKQALETVQALLPVLCQAHGLTPEQVVAIASNIGGKQALETVQALLPVLCQAHGLTP EQVVAIASNIGGKQALETVQALLPVLCQAHGLTPEQVVAIASNIGGKQALETVQALLPVLCQAHGLTPQ QVVAIASNNGGKQALETVQRLLPVLCQAHGLTPQQVVAIASNGGGKQALETVQRLLPVLCQAHGLTP EQVVAIASNIGGKQALETVQALLPVLCQAHGLTPEQVVAIASNIGGKQALETVQALLPVLCQAHGLTPQ QVVAIASNNGGKQALETVQRLLPVLCQAHGLTPEQVVAIASNIGGKQALETVQALLPVLCQAHGLTPQ QVVAIASNGGGKQALETVQRLLPVLCQAHGLTPQQVVAIASNGGGKQALETVQRLLPVLCQAHGLTP QQVVAIASNGGGRPALESIVAQLSRPDPALAALTNDHLVALACLGGRPALDAVKKGLGDPISRSQLVK SELEEKKSELRHKLKYVPHEYIELIEIARNSTQDRILEMKVMEFFMKVYGYRGKHLGGSRKPDGAIYTV GSPIDYGVIVDTKAYSGGYNLPIGQADEMQRYVEENQTRNKHINPNEWWKVYPSSVTEFKFLFVSGH FKGNYKAQLTRLNHITNCNGAVLSVEELLIGGEMIKAGTLTLEEVRRKFNNGEINFAAD |
| T002665 | hIDOL_Ex2A-L1 | NG-NG-NI-NG-NN-NN-HD-NG-NI-NI-NI-HD-HD-NG-NN-NG# | (SEQ ID NO:101) TTATGGCTAAAC CTGT# | (SEQ ID NO:43) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNGGGKQALETVQRLLPVLCQAHGLTPQQVVAI ASNGGGKQALETVQRLLPVLCQAHGLTPEQVVAIASNIGGKQALETVQALLPVLCQAHGLTPQQVVAI ASNGGGKQALETVQRLLPVLCQAHGLTPQQVVAIASNNGGKQALETVQRLLPVLCQAHGLTPQQVV AIASNNGGKQALETVQRLLPVLCQAHGLTPEQVVAIASHDGGKQALETVQRLLPVLCQAHGLTPQQV VAIASNGGGKQALETVQRLLPVLCQAHGLTPEQVVAIASNIGGKQALETVQALLPVLCQAHGLTPEQV VAIASNIGGKQALETVQALLPVLCQAHGLTPEQVVAIASNIGGKQALETVQALLPVLCQAHGLTPEQVV AIASHDGGKQALETVQRLLPVLCQAHGLTPEQVVAIASHDGGKQALETVQRLLPVLCQAHGLTPQQV VAIASNGGGKQALETVQRLLPVLCQAHGLTPQQVVAIASNNGGKQALETVQRLLPVLCQAHGLTPQQ VVAIASNGGGRPALESIVAQLSRPDPALAALTNDHLVALACLGGRPALDAVKKGLGDPISRSQLVKSEL EEKKSELRHKLKYVPHEYIELIEIARNSTQDRILEMKVMEFFMKVYGYRGKHLGGSRKPDGAIYTVGSP IDYGVIVDTKAYSGGYNLPIGQADEMQRYVEENQTRNKHINPNEWWKVYPSSVTEFKFLFVSGHFKG NYKAQLTRLNHITNCNGAVLSVEELLIGGEMIKAGTLTLEEVRRKFNNGEINFAAD |
| T002665 | hIDOL_Ex2A-R1 | NI-NN-HD-HD-HD-NI-NG-HD-HD-NI-NG-HD-NG-NN-HD-NG# | (SEQ ID NO:102) AGCCCATCCAT CTGCT# | (SEQ ID NO:44) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPEQVVAIASNIGGKQALETVQALLPVLCQAHGLTP QQVVAIASNNGGKQALETVQRLLPVLCQAHGLTPEQVVAIASHDGGKQALETVQRLLPVLCQAHGLT PEQVVAIASHDGGKQALETVQRLLPVLCQAHGLTPEQVVAIASHDGGKQALETVQRLLPVLCQAHGL TPEQVVAIASNIGGKQALETVQALLPVLCQAHGLTPQQVVAIASNGGGKQALETVQRLLPVLCQAHGL TPEQVVAIASHDGGKQALETVQRLLPVLCQAHGLTPEQVVAIASHDGGKQALETVQRLLPVLCQAHG LTPEQVVAIASNIGGKQALETVQALLPVLCQAHGLTPQQVVAIASNGGGKQALETVQRLLPVLCQAHG LTPEQVVAIASHDGGKQALETVQRLLPVLCQAHGLTPQQVVAIASNGGGKQALETVQRLLPVLCQAH GLTPQQVVAIASNNGGKQALETVQRLLPVLCQAHGLTPEQVVAIASHDGGKQALETVQRLLPVLCQA HGLTPQQVVAIASNGGGRPALESIVAQLSRPDPALAALTNDHLVALACLGGRPALDAVKKGLGDPISR SQLVKSELEEKKSELRHKLKYVPHEYIELIEIARNSTQDRILEMKVMEFFMKVYGYRGKHLGGSRKPD GAIYTVGSPIDYGVIVDTKAYSGGYNLPIGQADEMQRYVEENQTRNKHINPNEWWKVYPSSVTEFKFL FVSGHFKGNYKAQLTRLNHITNCNGAVLSVEELLIGGEMIKAGTLTLEEVRRKFNNGEINFAAD |
| T002666 | hIDOL_Ex3A-L1 | NN-HD-HD-NI-NI-NN-NN-NI-NN-HD-NG-HD-NG-HD-HD-NG# | (SEQ ID NO:103) GCCAAGGAGCT CTCCT# | (SEQ ID NO:45) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNNGGKQALETVQRLLPVLCQAHGLTPEQVVAI ASHDGGKQALETVQRLLPVLCQAHGLTPEQVVAIASHDGGKQALETVQRLLPVLCQAHGLTPEQVVA IASNIGGKQALETVQALLPVLCQAHGLTPEQVVAIASNIGGKQALETVQALLPVLCQAHGLTPQQVVAI ASNNGGKQALETVQRLLPVLCQAHGLTPQQVVAIASNNGGKQALETVQRLLPVLCQAHGLTPEQVVA IASNIGGKQALETVQALLPVLCQAHGLTPQQVVAIASNNGGKQALETVQRLLPVLCQAHGLTPEQVVAI ASHDGGKQALETVQRLLPVLCQAHGLTPQQVVAIASNGGGKQALETVQRLLPVLCQAHGLTPEQVVA IASHDGGKQALETVQRLLPVLCQAHGLTPQQVVAIASNGGGKQALETVQRLLPVLCQAHGLTPEQVV AIASHDGGKQALETVQRLLPVLCQAHGLTPEQVVAIASHDGGKQALETVQRLLPVLCQAHGLTPQQV VAIASNGGGRPALESIVAQLSRPDPALAALTNDHLVALACLGGRPALDAVKKGLGDPISRSQLVKSELE EKKSELRHKLKYVPHEYIELIEIARNSTQDRILEMKVMEFFMKVYGYRGKHLGGSRKPDGAIYTVGSPI DYGVIVDTKAYSGGYNLPIGQADEMQRYVEENQTRNKHINPNEWWKVYPSSVTEFKFLFVSGHFKG NYKAQLTRLNHITNCNGAVLSVEELLIGGEMIKAGTLTLEEVRRKFNNGEINFAAD |
| T002666 | hIDOL_Ex3A-R1 | NI-NG-NG-HD-NI-NI-HD-NI-NN-HD-HD-NG-HD-NI-HD-NG# | (SEQ ID NO:104) ATTCAACAGCCT CACT# | (SEQ ID NO:46) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPEQVVAIASNIGGKQALETVQALLPVLCQAHGLTP QQVVAIASNGGGKQALETVQRLLPVLCQAHGLTPQQVVAIASNGGGKQALETVQRLLPVLCQAHGLT PEQVVAIASHDGGKQALETVQRLLPVLCQAHGLTPEQVVAIASNIGGKQALETVQALLPVLCQAHGLT PEQVVAIASNIGGKQALETVQALLPVLCQAHGLTPEQVVAIASHDGGKQALETVQRLLPVLCQAHGLT PEQVVAIASNIGGKQALETVQALLPVLCQAHGLTPQQVVAIASNNGGKQALETVQRLLPVLCQAHGLT PEQVVAIASHDGGKQALETVQRLLPVLCQAHGLTPEQVVAIASHDGGKQALETVQRLLPVLCQAHGL TPQQVVAIASNGGGKQALETVQRLLPVLCQAHGLTPEQVVAIASHDGGKQALETVQRLLPVLCQAHG LTPEQVVAIASNIGGKQALETVQALLPVLCQAHGLTPEQVVAIASHDGGKQALETVQRLLPVLCQAHG LTPQQVVAIASNGGGRPALESIVAQLSRPDPALAALTNDHLVALACLGGRPALDAVKKGLGDPISRSQ LVKSELEEKKSELRHKLKYVPHEYIELIEIARNSTQDRILEMKVMEFFMKVYGYRGKHLGGSRKPDGAI YTVGSPIDYGVIVDTKAYSGGYNLPIGQADEMQRYVEENQTRNKHINPNEWWKVYPSSVTEFKFLFV SGHFKGNYKAQLTRLNHITNCNGAVLSVEELLIGGEMIKAGTLTLEEVRRKFNNGEINFAAD |
| T002667 | hANGPTL3_Ex1A-L1 | NI-NG-NG-NN-NG-NG-HD-HD-NG-HD-NG-NI-NN-NG-NG-NG# | (SEQ ID NO:105) ATTGTTCCTCTA GTTT# | (SEQ ID NO:47) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPEQVVAIASNIGGKQALETVQALLPVLCQAHGLTPQQVVAIA SNGGGKQALETVQRLLPVLCQAHGLTPQQVVAIASNGGGKQALETVQRLLPVLCQAHGLTPQQVVAI ASNNGGKQALETVQRLLPVLCQAHGLTPQQVVAIASNGGGKQALETVQRLLPVLCQAHGLTPQQVV AIASNGGGKQALETVQRLLPVLCQAHGLTPEQVVAIASHDGGKQALETVQRLLPVLCQAHGLTPEQV VAIASHDGGKQALETVQRLLPVLCQAHGLTPQQVVAIASNGGGKQALETVQRLLPVLCQAHGLTPEQ VVAIASHDGGKQALETVQRLLPVLCQAHGLTPQQVVAIASNGGGKQALETVQRLLPVLCQAHGLTPE QVVAIASNIGGKQALETVQALLPVLCQAHGLTPQQVVAIASNNGGKQALETVQRLLPVLCQAHGLTPQ QVVAIASNGGGKQALETVQRLLPVLCQAHGLTPQQVVAIASNGGGKQALETVQRLLPVLCQAHGLTP QQVVAIASNGGGRPALESIVAQLSRPDPALAALTNDHLVALACLGGRPALDAVKKGLGDPISRSQLVK SELEEKKSELRHKLKYVPHEYIELIEIARNSTQDRILEMKVMEFFMKVYGYRGKHLGGSRKPDGAIYTV GSPIDYGVIVDTKAYSGGYNLPIGQADEMQRYVEENQTRNKHINPNEWWKVYPSSVTEFKFLFVSGH FKGNYKAQLTRLNHITNCNGAVLSVEELLIGGEMIKAGTLTLEEVRRKFNNGEINFAAD |
| T002667 | hANGPTL3_Ex1A-R1 | NN-NI-NG-NN-NI-NI-NG-NG-NN-NG-HD-NG-NG-NN-NI-NG# | (SEQ ID NO:106) GATGAATTGTCT TGAT# | (SEQ ID NO:48) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PEQVVAIASNIGGKQALETVQALLPVLCQAHGLTPQQVVAIASNGGGKQALETVQRLLPVLCQAHGLT PQQVVAIASNNGGKQALETVQRLLPVLCQAHGLTPEQVVAIASNIGGKQALETVQALLPVLCQAHGLT PEQVVAIASNIGGKQALETVQALLPVLCQAHGLTPQQVVAIASNGGGKQALETVQRLLPVLCQAHGLT PQQVVAIASNGGGKQALETVQRLLPVLCQAHGLTPQQVVAIASNNGGKQALETVQRLLPVLCQAHGL TPQQVVAIASNGGGKQALETVQRLLPVLCQAHGLTPEQVVAIASHDGGKQALETVQRLLPVLCQAHG LTPQQVVAIASNGGGKQALETVQRLLPVLCQAHGLTPQQVVAIASNGGGKQALETVQRLLPVLCQAH GLTPQQVVAIASNNGGKQALETVQRLLPVLCQAHGLTPEQVVAIASNIGGKQALETVQALLPVLCQAH GLTPQQVVAIASNGGGRPALESIVAQLSRPDPALAALTNDHLVALACLGGRPALDAVKKGLGDPISRS QLVKSELEEKKSELRHKLKYVPHEYIELIEIARNSTQDRILEMKVMEFFMKVYGYRGKHLGGSRKPDG AIYTVGSPIDYGVIVDTKAYSGGYNLPIGQADEMQRYVEENQTRNKHINPNEWWKVYPSSVTEFKFLF VSGHFKGNYKAQLTRLNHITNCNGAVLSVEELLIGGEMIKAGTLTLEEVRRKFNNGEINFAAD |
| T002668 | hANGPTL3_Ex2A-L1 | HD-HD-NI-NN-NI-HD-NG-NG-NG-NG-NN-NG-NI-NN-NI-NG# | (SEQ ID NO:107) CCAGACTTTTGT AGAT# | (SEQ ID NO:49) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPEQVVAIASHDGGKQALETVQRLLPVLCQAHGLTPEQVVAI ASHDGGKQALETVQRLLPVLCQAHGLTPEQVVAIASNIGGKQALETVQALLPVLCQAHGLTPQQVVAI ASNNGGKQALETVQRLLPVLCQAHGLTPEQVVAIASNIGGKQALETVQALLPVLCQAHGLTPEQVVAI ASHDGGKQALETVQRLLPVLCQAHGLTPQQVVAIASNGGGKQALETVQRLLPVLCQAHGLTPQQVV AIASNGGGKQALETVQRLLPVLCQAHGLTPQQVVAIASNGGGKQALETVQRLLPVLCQAHGLTPQQV VAIASNGGGKQALETVQRLLPVLCQAHGLTPQQVVAIASNNGGKQALETVQRLLPVLCQAHGLTPQQ VVAIASNGGGKQALETVQRLLPVLCQAHGLTPEQVVAIASNIGGKQALETVQALLPVLCQAHGLTPQQ VVAIASNNGGKQALETVQRLLPVLCQAHGLTPEQVVAIASNIGGKQALETVQALLPVLCQAHGLTPQQ VVAIASNGGGRPALESIVAQLSRPDPALAALTNDHLVALACLGGRPALDAVKKGLGDPISRSQLVKSEL EEKKSELRHKLKYVPHEYIELIEIARNSTQDRILEMKVMEFFMKVYGYRGKHLGGSRKPDGAIYTVGSP IDYGVIVDTKAYSGGYNLPIGQADEMQRYVEENQTRNKHINPNEWWKVYPSSVTEFKFLFVSGHFKG NYKAQLTRLNHITNCNGAVLSVEELLIGGEMIKAGTLTLEEVRRKFNNGEINFAAD |
| T002668 | hANGPTL3_Ex2A-R1 | NN-NN-NI-NN-NI-NI-NN-NN-NG-HD-NG-NG-NG-NN-NI-NG# | (SEQ ID NO:108) GGAGAAGGTCT TTGAT# | (SEQ ID NO:50) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PQQVVAIASNNGGKQALETVQRLLPVLCQAHGLTPEQVVAIASNIGGKQALETVQALLPVLCQAHGLT PQQVVAIASNNGGKQALETVQRLLPVLCQAHGLTPEQVVAIASNIGGKQALETVQALLPVLCQAHGLT PEQVVAIASNIGGKQALETVQALLPVLCQAHGLTPQQVVAIASNNGGKQALETVQRLLPVLCQAHGLT PQQVVAIASNNGGKQALETVQRLLPVLCQAHGLTPQQVVAIASNGGGKQALETVQRLLPVLCQAHGL TPEQVVAIASHDGGKQALETVQRLLPVLCQAHGLTPQQVVAIASNGGGKQALETVQRLLPVLCQAHG LTPQQVVAIASNGGGKQALETVQRLLPVLCQAHGLTPQQVVAIASNGGGKQALETVQRLLPVLCQAH GLTPQQVVAIASNNGGKQALETVQRLLPVLCQAHGLTPEQVVAIASNIGGKQALETVQALLPVLCQAH GLTPQQVVAIASNGGGRPALESIVAQLSRPDPALAALTNDHLVALACLGGRPALDAVKKGLGDPISRS QLVKSELEEKKSELRHKLKYVPHEYIELIEIARNSTQDRILEMKVMEFFMKVYGYRGKHLGGSRKPDG AIYTVGSPIDYGVIVDTKAYSGGYNLPIGQADEMQRYVEENQTRNKHINPNEWWKVYPSSVTEFKFLF VSGHFKGNYKAQLTRLNHITNCNGAVLSVEELLIGGEMIKAGTLTLEEVRRKFNNGEINFAAD |
| T002669 | hPCSK9_Ex3A-L1 | NG-NN-HD-HD-HD-HD-NI-NG-NN-NG-HD-NN-NI-HD-NG-NG# | (SEQ ID NO:109) TGCCCCATGTC GACTT# | (SEQ ID NO:51) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNGGGKQALETVQRLLPVLCQAHGLTPQQVVAI ASNNGGKQALETVQRLLPVLCQAHGLTPEQVVAIASHDGGKQALETVQRLLPVLCQAHGLTPEQVVA IASHDGGKQALETVQRLLPVLCQAHGLTPEQVVAIASHDGGKQALETVQRLLPVLCQAHGLTPEQVV AIASHDGGKQALETVQRLLPVLCQAHGLTPEQVVAIASNIGGKQALETVQALLPVLCQAHGLTPQQVV AIASNGGGKQALETVQRLLPVLCQAHGLTPQQVVAIASNNGGKQALETVQRLLPVLCQAHGLTPQQV VAIASNGGGKQALETVQRLLPVLCQAHGLTPEQVVAIASHDGGKQALETVQRLLPVLCQAHGLTPQQ VVAIASNNGGKQALETVQRLLPVLCQAHGLTPEQVVAIASNIGGKQALETVQALLPVLCQAHGLTPEQ VVAIASHDGGKQALETVQRLLPVLCQAHGLTPQQVVAIASNGGGKQALETVQRLLPVLCQAHGLTPQ QVVAIASNGGGRPALESIVAQLSRPDPALAALTNDHLVALACLGGRPALDAVKKGLGDPISRSQLVKS ELEEKKSELRHKLKYVPHEYIELIEIARNSTQDRILEMKVMEFFMKVYGYRGKHLGGSRKPDGAIYTVG SPIDYGVIVDTKAYSGGYNLPIGQADEMQRYVEENQTRNKHINPNEWWKVYPSSVTEFKFLFVSGHF KGNYKAQLTRLNHITNCNGAVLSVEELLIGGEMIKAGTLTLEEVRRKFNNGEINFAAD |
| T002669 | hPCSK9_Ex3A-R1 | HD-NG-NN-NN-NN-HD-NI-NI-NI-NN-NI-HD-NI-NN-NI-NG# | (SEQ ID NO:110) CTGGGCAAAGA CAGAT# | (SEQ ID NO:52) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPEQVVAIASHDGGKQALETVQRLLPVLCQAHGLT PQQVVAIASNGGGKQALETVQRLLPVLCQAHGLTPQQVVAIASNNGGKQALETVQRLLPVLCQAHGL TPQQVVAIASNNGGKQALETVQRLLPVLCQAHGLTPQQVVAIASNNGGKQALETVQRLLPVLCQAHG LTPEQVVAIASHDGGKQALETVQRLLPVLCQAHGLTPEQVVAIASNIGGKQALETVQALLPVLCQAHG LTPEQVVAIASNIGGKQALETVQALLPVLCQAHGLTPEQVVAIASNIGGKQALETVQALLPVLCQAHGL TPQQVVAIASNNGGKQALETVQRLLPVLCQAHGLTPEQVVAIASNIGGKQALETVQALLPVLCQAHGL TPEQVVAIASHDGGKQALETVQRLLPVLCQAHGLTPEQVVAIASNIGGKQALETVQALLPVLCQAHGL TPQQVVAIASNNGGKQALETVQRLLPVLCQAHGLTPEQVVAIASNIGGKQALETVQALLPVLCQAHGL TPQQVVAIASNGGGRPALESIVAQLSRPDPALAALTNDHLVALACLGGRPALDAVKKGLGDPISRSQL VKSELEEKKSELRHKLKYVPHEYIELIEIARNSTQDRILEMKVMEFFMKVYGYRGKHLGGSRKPDGAIY TVGSPIDYGVIVDTKAYSGGYNLPIGQADEMQRYVEENQTRNKHINPNEWWKVYPSSVTEFKFLFVS GHFKGNYKAQLTRLNHITNCNGAVLSVEELLIGGEMIKAGTLTLEEVRRKFNNGEINFAAD |
| T002670 | hPCSK9_Ex12A-L1 | NN-NN-HD-NI-NN-NN-NG-NN-NI-HD-HD-NN-NG-NN-NN-NG# | (SEQ ID NO:111) GGCAGGTGACC GTGGT# | (SEQ ID NO:53) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNNGGKQALETVQRLLPVLCQAHGLTPQQVVAI ASNNGGKQALETVQRLLPVLCQAHGLTPEQVVAIASHDGGKQALETVQRLLPVLCQAHGLTPEQVVA IASNIGGKQALETVQALLPVLCQAHGLTPQQVVAIASNNGGKQALETVQRLLPVLCQAHGLTPQQVVA IASNNGGKQALETVQRLLPVLCQAHGLTPQQVVAIASNGGGKQALETVQRLLPVLCQAHGLTPQQVV AIASNNGGKQALETVQRLLPVLCQAHGLTPEQVVAIASNIGGKQALETVQALLPVLCQAHGLTPEQVV AIASHDGGKQALETVQRLLPVLCQAHGLTPEQVVAIASHDGGKQALETVQRLLPVLCQAHGLTPQQV VAIASNNGGKQALETVQRLLPVLCQAHGLTPQQVVAIASNGGGKQALETVQRLLPVLCQAHGLTPQQ VVAIASNNGGKQALETVQRLLPVLCQAHGLTPQQVVAIASNNGGKQALETVQRLLPVLCQAHGLTPQ QVVAIASNGGGRPALESIVAQLSRPDPALAALTNDHLVALACLGGRPALDAVKKGLGDPISRSQLVKS ELEEKKSELRHKLKYVPHEYIELIEIARNSTQDRILEMKVMEFFMKVYGYRGKHLGGSRKPDGAIYTVG SPIDYGVIVDTKAYSGGYNLPIGQADEMQRYVEENQTRNKHINPNEWWKVYPSSVTEFKFLFVSGHF KGNYKAQLTRLNHITNCNGAVLSVEELLIGGEMIKAGTLTLEEVRRKFNNGEINFAAD |
| T002670 | hPCSK9_Ex12A-R1 | NN-HD-NI-NN-HD-HD-NI-NN-NG-HD-NI-NN-NN-NN-NG-NG# | (SEQ ID NO:112) GCAGCCAGTCA GGGTT# | (SEQ ID NO:54) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PEQVVAIASHDGGKQALETVQRLLPVLCQAHGLTPEQVVAIASNIGGKQALETVQALLPVLCQAHGLT PQQVVAIASNNGGKQALETVQRLLPVLCQAHGLTPEQVVAIASHDGGKQALETVQRLLPVLCQAHGL TPEQVVAIASHDGGKQALETVQRLLPVLCQAHGLTPEQVVAIASNIGGKQALETVQALLPVLCQAHGL TPQQVVAIASNNGGKQALETVQRLLPVLCQAHGLTPQQVVAIASNGGGKQALETVQRLLPVLCQAHG LTPEQVVAIASHDGGKQALETVQRLLPVLCQAHGLTPEQVVAIASNIGGKQALETVQALLPVLCQAHG LTPQQVVAIASNNGGKQALETVQRLLPVLCQAHGLTPQQVVAIASNNGGKQALETVQRLLPVLCQAH GLTPQQVVAIASNNGGKQALETVQRLLPVLCQAHGLTPQQVVAIASNGGGKQALETVQRLLPVLCQA HGLTPQQVVAIASNGGGRPALESIVAQLSRPDPALAALTNDHLVALACLGGRPALDAVKKGLGDPISR SQLVKSELEEKKSELRHKLKYVPHEYIELIEIARNSTQDRILEMKVMEFFMKVYGYRGKHLGGSRKPD GAIYTVGSPIDYGVIVDTKAYSGGYNLPIGQADEMQRYVEENQTRNKHINPNEWWKVYPSSVTEFKFL FVSGHFKGNYKAQLTRLNHITNCNGAVLSVEELLIGGEMIKAGTLTLEEVRRKFNNGEINFAAD |
| T002672 | hPCSK9_Ex3_AN-L1 | NG-NN-HD-HD-HD-HD-NI-NG-NN-NG-N-NN-NI-HD-NG-NG# | (SEQ ID NO:113) TGCCCCATGT5 GACTT# | (SEQ ID NO:55) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNGGGKQALETVQRLLPVLCQAHGLTPQQVVAI ASNNGGKQALETVQRLLPVLCQAHGLTPEQVVAIASHDGGKQALETVQRLLPVLCQAHGLTPEQVVA IASHDGGKQALETVQRLLPVLCQAHGLTPEQVVAIASHDGGKQALETVQRLLPVLCQAHGLTPEQVV AIASHDGGKQALETVQRLLPVLCQAHGLTPEQVVAIASNIGGKQALETVQALLPVLCQAHGLTPQQVV AIASNGGGKQALETVQRLLPVLCQAHGLTPQQVVAIASNNGGKQALETVQRLLPVLCQAHGLTPQQV VAIASNGGGKQALETVQRLLPVLCQAHGLTPQQVVAIASNGGKQALETVQRLLPVLCQAHGLTPQQV VAIASNNGGKQALETVQRLLPVLCQAHGLTPEQVVAIASNIGGKQALETVQALLPVLCQAHGLTPEQV VAIASHDGGKQALETVQRLLPVLCQAHGLTPQQVVAIASNGGGKQALETVQRLLPVLCQAHGLTPQQ VVAIASNGGGRPALESIVAQLSRPDPALAALTNDHLVALACLGGRPALDAVKKGLGDPISRSQLVKSEL EEKKSELRHKLKYVPHEYIELIEIARNSTQDRILEMKVMEFFMKVYGYRGKHLGGSRKPDGAIYTVGSP IDYGVIVDTKAYSGGYNLPIGQADEMQRYVEENQTRNKHINPNEWWKVYPSSVTEFKFLFVSGHFKG NYKAQLTRLNHITNCNGAVLSVEELLIGGEMIKAGTLTLEEVRRKFNNGEINFAAD |
| T002672 | hPCSK9_Ex3A-R1 | HD-NG-NN-NN-NN-HD-NI-NI-NI-NN-NI-HD-NI-NN-NI-NG# | (SEQ ID NO:114) CTGGGCAAAGA CAGAT# | (SEQ ID NO:56) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPEQVVAIASHDGGKQALETVQRLLPVLCQAHGLT PQQVVAIASNGGGKQALETVQRLLPVLCQAHGLTPQQVVAIASNNGGKQALETVQRLLPVLCQAHGL TPQQVVAIASNNGGKQALETVQRLLPVLCQAHGLTPQQVVAIASNNGGKQALETVQRLLPVLCQAHG LTPEQVVAIASHDGGKQALETVQRLLPVLCQAHGLTPEQVVAIASNIGGKQALETVQALLPVLCQAHG LTPEQVVAIASNIGGKQALETVQALLPVLCQAHGLTPEQVVAIASNIGGKQALETVQALLPVLCQAHGL TPQQVVAIASNNGGKQALETVQRLLPVLCQAHGLTPEQVVAIASNIGGKQALETVQALLPVLCQAHGL TPEQVVAIASHDGGKQALETVQRLLPVLCQAHGLTPEQVVAIASNIGGKQALETVQALLPVLCQAHGL TPQQVVAIASNNGGKQALETVQRLLPVLCQAHGLTPEQVVAIASNIGGKQALETVQALLPVLCQAHGL TPQQVVAIASNGGGRPALESIVAQLSRPDPALAALTNDHLVALACLGGRPALDAVKKGLGDPISRSQL VKSELEEKKSELRHKLKYVPHEYIELIEIARNSTQDRILEMKVMEFFMKVYGYRGKHLGGSRKPDGAIY TVGSPIDYGVIVDTKAYSGGYNLPIGQADEMQRYVEENQTRNKHINPNEWWKVYPSSVTEFKFLFVS GHFKGNYKAQLTRLNHITNCNGAVLSVEELLIGGEMIKAGTLTLEEVRRKFNNGEINFAAD |
| T002673 | hPCSK9_Ex12A-R1 | NN-HD-NI-NN-HD-HD-NI-NN-NG-HD-NI-NN-NN-NN-NG-NG# | (SEQ ID NO:115) GCAGCCAGTCA GGGTT# | (SEQ ID NO:57) MGDPKKKRKVIDKETAAAKFERQHMDSIDIADLRTLGYSQQQQEKIKPKVRSTVAQHHEALVGHGFT HAHIVALSQHPAALGTVAVKYQDMIAALPEATHEAIVGVGKQWSGARALEALLTVAGELRGPPLQLDT GQLLKIAKRGGVTAVEAVHAWRNALTGAPLNLTPQQVVAIASNNGGKQALETVQRLLPVLCQAHGLT PEQVVAIASHDGGKQALETVQRLLPVLCQAHGLTPEQVVAIASNIGGKQALETVQALLPVLCQAHGLT PQQVVAIASNNGGKQALETVQRLLPVLCQAHGLTPEQVVAIASHDGGKQALETVQRLLPVLCQAHGL TPEQVVAIASHDGGKQALETVQRLLPVLCQAHGLTPEQVVAIASNIGGKQALETVQALLPVLCQAHGL TPQQVVAIASNNGGKQALETVQRLLPVLCQAHGLTPQQVVAIASNGGGKQALETVQRLLPVLCQAHG LTPEQVVAIASHDGGKQALETVQRLLPVLCQAHGLTPEQVVAIASNIGGKQALETVQALLPVLCQAHG LTPQQVVAIASNNGGKQALETVQRLLPVLCQAHGLTPQQVVAIASNNGGKQALETVQRLLPVLCQAH GLTPQQVVAIASNNGGKQALETVQRLLPVLCQAHGLTPQQVVAIASNGGGKQALETVQRLLPVLCQA HGLTPQQVVAIASNGGGRPALESIVAQLSRPDPALAALTNDHLVALACLGGRPALDAVKKGLGDPISR SQLVKSELEEKKSELRHKLKYVPHEYIELIEIARNSTQDRILEMKVMEFFMKVYGYRGKHLGGSRKPD GAIYTVGSPIDYGVIVDTKAYSGGYNLPIGQADEMQRYVEENQTRNKHINPNEWWKVYPSSVTEFKFL FVSGHFKGNYKAQLTRLNHITNCNGAVLSVEELLIGGEMIKAGTLTLEEVRRKFNNGEINFAAD |
| T002673 | hPCSK9Ex12_AN-L1 | NN-NN-HD-NI-NN-NN-NG-NN-NI-HD-N-NN-NG-NN-NN-NG# | (SEQ ID NO:116) GGCAGGTGAC5 GTGGT# | (SEQ ID NO:58) MGDPKKKRKVIDYPYDVPDYAIDIADLRTLGYSQQQQEKIKPKVRSTVAQHHEALVGHGFTHAHIVAL SQHPAALGTVAVKYQDMIAALPEATHEAIVGVGKQWSGARALEALLTVAGELRGPPLQLDTGQLLKIA KRGGVTAVEAVHAWRNALTGAPLNLTPQQVVAIASNNGGKQALETVQRLLPVLCQAHGLTPQQVVAI ASNNGGKQALETVQRLLPVLCQAHGLTPEQVVAIASHDGGKQALETVQRLLPVLCQAHGLTPEQVVA IASNIGGKQALETVQALLPVLCQAHGLTPQQVVAIASNNGGKQALETVQRLLPVLCQAHGLTPQQVVA IASNNGGKQALETVQRLLPVLCQAHGLTPQQVVAIASNGGGKQALETVQRLLPVLCQAHGLTPQQVV AIASNNGGKQALETVQRLLPVLCQAHGLTPEQVVAIASNIGGKQALETVQALLPVLCQAHGLTPEQVV AIASHDGGKQALETVQRLLPVLCQAHGLTPQQVVAIASNGGKQALETVQRLLPVLCQAHGLTPQQVV AIASNNGGKQALETVQRLLPVLCQAHGLTPQQVVAIASNGGGKQALETVQRLLPVLCQAHGLTPQQV VAIASNNGGKQALETVQRLLPVLCQAHGLTPQQVVAIASNNGGKQALETVQRLLPVLCQAHGLTPQQ VVAIASNGGGRPALESIVAQLSRPDPALAALTNDHLVALACLGGRPALDAVKKGLGDPISRSQLVKSEL EEKKSELRHKLKYVPHEYIELIEIARNSTQDRILEMKVMEFFMKVYGYRGKHLGGSRKPDGAIYTVGSP IDYGVIVDTKAYSGGYNLPIGQADEMQRYVEENQTRNKHINPNEWWKVYPSSVTEFKFLFVSGHFKG NYKAQLTRLNHITNCNGAVLSVEELLIGGEMIKAGTLTLEEVRRKFNNGEINFAAD |

## Claims

1. A method for encapsulating a RNA molecule encoding a TALE-Nuclease, comprising the steps of
a) Engineering a RNA molecule encoding a TALE-Nuclease;
b) Complexing the RNA molecule encoding said TALE-Nuclease with at least one biodegradable matrix comprising at least a core hydrophobic domain and a proximal polar domain to favor interactions with water molecules;
c) Forming particles encapsulating said RNA molecule encoding said TALE-Nuclease of 50 to 100 nm diameter range;
wherein said TALE-Nuclease binds a target sequence selected from SEQ ID NO: 59 to 78.

2. A method according to claim 1, wherein said core hydrophobic domain is a biodegradable conjugate of hydrophobic monomers.

3. A method according to claim 2, wherein said hydrophobic monomers conjugate are of aminolipids, such as ionized cationic lipid 1,2-dilinoleyloxy-3-dimethylaminopropane (DLinDMA).

4. A method according to claim 3, wherein said aminolipids are mixed with PEG-lipids to form said polar domain.

5. A method according to claim 3 or 4, wherein said aminolipids allows binding of ApoE in-vivo, said ApoE facilitating Apo E mediated endocytosis.

6. A method according to any one of claims 1 to 5, wherein said at least one polar domain is poly-N,N-di(C1-C6)alkyl-amino(C1-C6)alkyl-ethacrylate, poly-N,N-di(C1-C6)alkylamino(C1-C6)alkyl-methacrylate, or poly-N,N-di(C1-C6)alkyl-amino(C1-C6)alkyl-acrylate, or a combination thereof.

7. A method according to claim 6, wherein said hydrophobic monomers include at least (C2-C8)alkyl- ethacrylate, a (C2-C8)alkyl-methacrylate, or a (C2-C8)alkyl-acrylate.

8. A method according to any one of claims 1 to 7, wherein said hydrophobic monomers conjugate are mixed with carboxylic acid monomers and tertiary amino monomers.

9. A method according to any one of claims 1 to 8, wherein said at least one polar domain is linked to a targeting domain.

10. A method according to any one of claims 1 to 9, wherein said at least one polar domain is linked to a N-acetylgalactosamine ligand.

11. A biodegradable delivery capsule obtainable by the method according to any one of claims 1 to 10.

12. A biodegradable delivery capsule, **characterized in that** a RNA molecule encoding a TALE-Nuclease is complexed with at least one polar domain, which is linked to biodegradable conjugate(s) of hydrophobic monomers to form spherical particles of 50 to 100 nm diameter range, said TALE-Nuclease binding a target sequence selected from SEQ ID NO: 59 to 78; for use in the treatment of a liver disease caused by hepatitis B virus (HBV), such as hepatitis or liver cancer.

13. A biodegradable delivery capsule for use according to claim 12, wherein said biodegradable matrix comprises at least two polar domains, such that the inner core particle is hydrophilic.

14. A pharmaceutical composition comprising a biodegradable delivery system according to any one of claims 11 to 13 with a pharmaceutically injectable medium.

15. A pharmaceutical composition according to claim 14, for use in the treatment of a liver disease caused by hepatitis B virus (HBV), such as hepatitis or liver cancer.

## Patentansprüche

1. Verfahren zum Einkapseln eines RNA-Moleküls, das für eine TALE-Nuklease kodiert, umfassend die Schritte:
a) Engineering eines RNA-Moleküls, das für eine TALE-Nuklease kodiert;
b) Komplexieren des RNA-Moleküls, das für die TALE-Nuklease codiert, mit mindestens einer biologisch abbaubaren Matrix, die mindestens eine hydrophobe Kerndomäne und eine proximale polare Domäne umfasst, um Wechselwirkungen mit Wassermolekülen zu begünstigen;
c) Bilden von Partikeln, die das für die TALE-Nuklease kodierende RNA-Molekül, einkapseln, mit einem Durchmesserbereich von 50 bis 100 nm:
wobei die TALE-Nuklease eine aus SEQ ID NO: 59 bis 78 ausgewählte Zielsequenz bindet.

2. Verfahren nach Anspruch 1, wobei die hydrophobe Kerndomäne ein biologisch abbaubares Konjugat aus hydrophoben Monomeren ist.

3. Verfahren nach Anspruch 2, wobei das Konjugat aus hydrophoben Monomeren aus Aminolipiden, wie ionisiertem kationischem Lipid-1,2-Dilinoleyloxy-3-dimethylaminopropan (DLindmA), besteht.

4. Verfahren nach Anspruch 3, wobei die Aminolipide mit PEG-Lipiden gemischt werden, um die polare Domäne zu bilden.

5. Verfahren nach Anspruch 3 oder 4, wobei die Aminolipide die Bindung von APoE in vivo ermöglichen, wobei das APoE die ApoE-vermittelte Endozytose erleichtert.

6. Verfahren nach einem der Ansprüche 1 bis 5, wobei die mindestens eine polare Domäne Poly-N,N-di(C1-C6)alkyl-amino(C1-C6)alkyl-ethacrylat, Poly-N,N-di(C1-C6)alkyl-amino(C1-C6)alkyl-methacrylat oder Poly-N,N-di(C1-C6)alkyl-amino(C1-C6)alkyl-acrylat oder eine Kombination davon ist.

7. Verfahren nach Anspruch 6, wobei die hydrophoben Monomere mindestens (C2-C8)-Alkylethacrylat, ein (C2-C8)-Alkylmethacrylat oder ein (C2-C8)-Alkylacrylat umfassen.

8. Verfahren nach einem der Ansprüche 1 bis 7, wobei das hydrophobe Monomer-Konjugat mit Carbonsäuremonomeren und tertiären Aminomonomeren gemischt wird.

9. Verfahren nach einem der Ansprüche 1 bis 8, wobei die mindestens eine polare Domäne mit einer Targeting-Domäne verbunden ist.

10. Verfahren nach einem der Ansprüche 1 bis 9, wobei die mindestens eine polare Domäne mit einem N-Acetylgalactosamin-Liganden verbunden ist.

11. Biologisch abbaubare Abgabekapsel, die durch das Verfahren nach einem der Ansprüche 1 bis 10 erhältlich ist.

12. Biologisch abbaubare Abgabekapsel, **dadurch gekennzeichnet, dass** ein für eine TALE-Nuklease kodierendes RNA-Molekül mit mindestens einer polaren Domäne komplexiert wird, die mit (einem) biologisch abbaubaren Konjugat(en) aus hydrophoben Monomeren verbunden ist, um kugelförmige Partikel mit einem Durchmesser im Bereich von 50 bis 100 nm zu bilden, wobei die TALE-Nuklease eine aus SEQ ID NO: 59 bis 78 ausgewählte Zielsequenz bindet; zur Verwendung bei der Behandlung einer durch das Hepatitis-B-Virus (HBV) verursachten Lebererkrankung, wie Hepatitis oder Leberkrebs.

13. Biologisch abbaubare Abgabekapsel zur Verwendung nach Anspruch 12, wobei die biologisch abbaubare Matrix mindestens zwei polare Domänen umfasst, so dass das innere Kernpartikel hydrophil ist.

14. Pharmazeutische Zusammensetzung, umfassend ein biologisch abbaubares Abgabesystem nach einem der Ansprüche 11 bis 13 mit einem pharmazeutisch injizierbaren Medium.

15. Pharmazeutische Zusammensetzung nach Anspruch 14 zur Verwendung bei der Behandlung einer durch das Hepatitis-B-Virus (HBV) verursachten Lebererkrankung, wie Hepatitis oder Leberkrebs.

## Revendications

1. Procédé d'encapsulation d'une molécule d'ARN codant pour une nucléase TALE, comprenant les étapes de :
a) l'ingénierie d'une molécule d'ARN codant pour une nucléase TALE ;
b) la complexation de la molécule d'ARN codant ladite nucléase TALE avec au moins une matrice biodégradable comprenant au moins un domaine hydrophobe central et un domaine polaire proximal pour favoriser des interactions avec des molécules d'eau ;
c) la formation de particules encapsulant ladite molécule d'ARN codant ladite nucléase TALE d'une plage de 50 à 100 nm de diamètre :
dans lequel ladite nucléase TALE se lie à une séquence cible choisie parmi SEQ ID NO: 59 à 78.

2. Procédé selon la revendication 1, dans lequel ledit domaine hydrophobe central est un conjugué biodégradable de monomères hydrophobes.

3. Procédé selon la revendication 2, dans lequel ledit conjugué de monomères hydrophobes est d'aminolipides, tels que le lipide cationique ionisé 1,2-dilino-leyloxy-3-diméthylaminopropane (DLinDMA).

4. Procédé selon la revendication 3, dans lequel lesdits aminolipides sont mélangés avec des PEG-lipides pour former ledit domaine polaire.

5. Procédé selon la revendication 3 et 4, dans lequel lesdits aminolipides permettent la liaison d'ApoE in vivo, ladite ApoE facilitant l'endocytose médiée par l'ApoE.

6. Procédé selon l'une quelconque des revendications 1 à 5, dans lequel ledit au moins un domaine polaire est un poly-N,N-di(C1 -C6)alkyl-amino(C1 - C6)alkyl-éthacrylate, un poly-N,N-di(C1 -C6)alkyl-amino(C1 -C6)alkyl-méthacrylate, ou un poly-N,N-di(C1 -C6)alkyl-amino(C1 -C6)alkyl-acrylate, ou une combinaison de ceux-ci.

7. Procédé selon la revendication 6, dans lequel lesdits monomères hydrophobes comprennent au moins un (C2-C8)alkyl-éthacrylate, un (C2-C8)alkyl-méthacrylate ou un (C2-C8)alkyl-acrylate.

8. Procédé selon l'une quelconque des revendications 1 à 7, dans lequel ledit conjugué de monomères hydrophobes est mélangé avec des monomères d'acide carboxylique et des monomères amino tertiaires.

9. Procédé selon l'une quelconque des revendications 1 à 8, dans lequel ledit au moins un domaine polaire est lié à un domaine de ciblage.

10. Procédé selon l'une quelconque des revendications 1 à 9, dans lequel ledit au moins un domaine polaire est lié à un ligand de N-acétylgalactosamine.

11. Capsule d'administration biodégradable pouvant être obtenue par le procédé selon l'une quelconque des revendications 1 à 10.

12. Capsule d'administration biodégradable, **caractérisée en ce qu'**une molécule d'ARN codant pour une nucléase TALE est complexée avec au moins un domaine polaire, qui est lié à un ou plusieurs conjugué(s) biodégradables de monomères hydrophobes pour former des particules sphériques d'une plage de diamètre de 50 à 100 nm, ladite nucléase TALE se liant à une séquence cible choisie parm SEQ ID NO: 59 à 78 ; destinée à être utilisée dans le traitement d'une maladie hépatique causée par le virus de l'hépatite B (VHB), tel que l'hépatite ou le cancer du foie.

13. Capsule d'administration biodégradable pour l'usage selon la revendication 12, dans laquelle ladite matrice biodégradable comprend au moins deux domaines polaires, de sorte que la particule de noyau interne est hydrophile.

14. Composition pharmaceutique comprenant un système d'administration biodégradable selon l'une quelconque des revendications 11 à 13 avec un milieu pharmaceutiquement injectable.

15. Composition pharmaceutique selon la revendication 14, destinée à être utilisée dans le traitement d'une maladie hépatique causée par le virus de l'hépatite B (VHB), tel que l'hépatite ou le cancer du foie.
